(19) Europäisches Patentamt
European Patent Office
Office européen des brevets

(11) EP 1 158 447 A1

# (12) EUROPEAN PATENT APPLICATION

(43) Date of publication:
**28.11.2001 Bulletin 2001/48**

(51) Int Cl.$^7$: **G06F 19/00**

(21) Application number: **00111352.1**

(22) Date of filing: **26.05.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **GMD- Forschungszentrum Informationstechnik GmbH**
**53754 St. Augustin (DE)**

(72) Inventors:
- **Zimmer, Ralf, Dr.**
**53115 Bonn (DE)**
- **Küffner, Robert, Dr.**
**53229 Bonn (DE)**
- **Zien, Alexander**
**53913 Swisttal (DE)**

(74) Representative:
**Meyers, Hans-Wilhelm, Dr.Dipl.-Chem. et al**
**Patentanwälte**
**von Kreisler-Selting-Werner**
**Postfach 10 22 41**
**50462 Köln (DE)**

## (54) Method for evaluating states of biological systems

(57) A method for evaluating states of biological systems comprising the steps of

a) constructing a pathway comprising at least two molecules and their interaction network,

b) measuring expression data with an appropriate experiment and measuring device and

c) calculating a score for said pathway based on said experimental quantification of the amounts of molecules in said system, said score indicating an intensity of realization of said pathway in said state of said biological system.

Figure1:

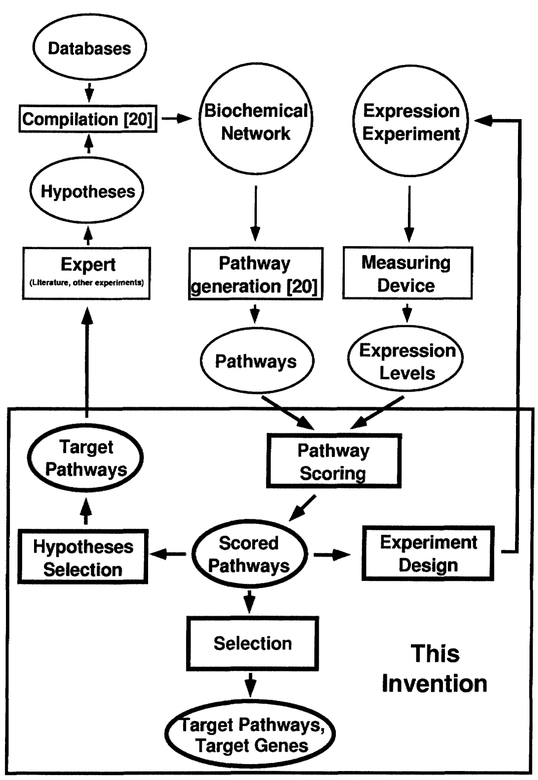




Printed by Jouve, 75001 PARIS (FR)

## Description

The present invention relates to a method for evaluating states of biological systems.

Background of the Invention

In the last years, it has become possible to measure and quantify abundances of molecules in biological systems on a large scale. Most frequently, the measured molecules are mRNAs (e.g. with DNA chips or micro-arrays) or proteins (e.g. via mass spectrometry). Such measurements can now be performed by several established techniques, including

- EST sequencing, clustering and counting [26, 27];
- micro-arrays [10, 14, 15, 23, 30, 33];
- DNA-chips, [7, 22].

Several methods have been proposed in order to interpret the large amounts of data gathered by the above means. They include manual interpretation [10, 16] sometimes aided by computer visualization [6, 24] that often builds on clustering [12, 36]. Coarse-grain function predictions are being performed semi-automatically as an extension of cluster analysis [8], for a particular group of genes with unique features that can be exploited by specialized search algorithms [35] and using supervised machine learning techniques [5].

All of the automatic methods utilize a rather broad and diffuse notion of biological function. No method, possibly except for human expertise, integrates the detailed knowledge about parts of the biological networks into the evaluation of gene expression data yet. The clustering methods do not explicitly exploit functional knowledge.

There are methods published that try to make use of knowledge on metabolic pathways for the interpretation of gene expression data e.g. [13]. In these methods, genes are clustered according to the expression time series. The clusters are used to restrict the sets of possible reactions. From these restricted sets, pathways are constructed systematically as described in [17], similar to [20]. The result is long list of quite similar pathways, as is said in [13]. Neither does the method provide a measure for discriminating between these pathways, nor does it make it more likely that the pathways generated are realized in the cells under investigation. When this method is applied starting from some expression level based clustering of the genes, a subset of all possible partial nets is generated that contains some (but not all) similarly regulated subsets of genes. This approach has several disadvantages: the subsets in general do not correspond to complete pathways, but, by definition, only well-correlated sets of genes are found, a reasonable null-model is not exploited to detect significant subsets among those with similar characteristic, and some pathways that can be recognized as being realized from the expression data cannot be found, as will be shown in the worked out example below.

*Large Scale Measurements*

Such measurements (often called differential display) of molecular differences of cells are state-of-the-art on a large scale (several thousand genes or gene products) or even on the level of complete genomes or the whole inventory of genes available to an organism in specific cell states. One method used today for such measurements [1] is to specifically fabricate DNA chips containing short DNA fragments (oligo-nucleotides) complementary to the genes of interest in a cell probe.

Another method (DNA arrays) is to attach (print) small amounts of each DNA fragment to a array-like arrangement on glass slides or nylon membranes and to hybridize the probe DNA with all the attached fragment samples simultaneously. Although the human genome has not been completely sequenced and although all human genes have not yet been identified, DNA chip technology already allows for a complete screen of human genes on one chip: current technology allows to fabricate chips with several hundred-thousand oligonucleotides, which could cover the 100.000 to 140.000 human genes two to three-fold [7]. DNA arrays are in use, which hold fragments from each of the 6000 genes of the eucaryotic organism yeast.

*Analysis of Measurements*

The major problem of such measurements is the evaluation of the large number of expression levels measured: usually a differential display with a large DNA chip results in several thousand differences of different confidence. The evaluation of these differences is additionally hindered by the current limitations of the accuracy of measurements

on such chips or arrays, which makes individual expression levels quite unreliable. With state-of-the-art techniques, a two to three-fold increase or decrease in expression level is considered to be a real increase or decrease rather than a measuring error. One obvious possibility is to use these measurements to determine the subset of those genes that are truly differently expressed and, thus, are related to the phenotypical differences of the compared states. The subsequent tasks are to exhibit why and how this subset could explain the causes and consequences of these differences. In the near future these two questions have to be answered for many such experiments, as the experiments will be performed for large numbers of cell states, and the correct and fast answer to these questions for many experiments will be of direct scientific, pharmaceutical, and crucial commercial importance and value for companies striving to find new innovative treatments for diseases. This implies that the evaluation of such experimental data has to be done in large parts with automated computer methods that have been validated and calibrated.

*Metabolic Pathways and Petri Nets*

In particular time series measurements of systems, i.e. measuring the expression of a large set of - sometimes even all - expressed genes for a number of subsequent time intervals, allow to analyze the detailed interaction in known pathways as well as to infer new putative relations. Methods have been developed [20] which allow to represent metabolic and regulatory network with suitable graph-like structures, e.g. so called Petri nets [29, 32], and to enumerate all possible pathways from the database of known chemical reactions performed by organisms. Pathways can be confined to lead from some definable set of starting molecular units (the reactants) to another definable set of units (the products). Valid pathways can be defined to account for additional biological knowledge and to exclude biologically impossible paths in order to substantially restrict the number of all possible paths in the interaction network.

Summary of the invention

The present invention discloses a method for evaluating states of biological systems comprising the steps of

a) constructing a pathway comprising at least two molecules and their interaction network and

b) measuring expression data with an appropriate experiment and measuring device

c) calculating a score for said pathway based on the experimental quantification of the amounts of molecules in said system, said score indicating an intensity of realization of said pathway in said state of said biological system.

Pathways as used herein are structures that are suitable to describe relevant aspects of some molecules and their interactions. In a preferred embodiment, pathways are minimal substructures of complete representations of cells that still cover a biologically important process. An example are closed pathways as described in [20].

The invention involves a procedure to evaluate biological and genomic data, specifically measurements of the quantitative abundances of sets of molecules in specific cell states. Here abundances are estimated or measured as concentrations or expression levels represented as numbers meaning either absolute counts or relative differences as compared to some reference state.

Suitable molecules are e.g. substrates, small molecules, drug molecules, genes, DNA sequences, mRNA molecules, pre-proteins, or proteins.

It is known that the comparison of such states, especially the expression levels of genes in such states, can yield important information on the differences of different cells on the molecular level. Of great importance is the comparison of diseased and normal cells in order to exhibit or detect the causes and consequences of diseases with the final goal of finding possible target genes for drug treatment to remedy a disease or relieve its symptoms. Another important application is the investigation of the response of specific cells to treatment with potential drugs in order to assess their efficacy or toxicity.

In a preferred embodiment, the method of the invention is followed by an estimation of the significance of said score of said pathway by the steps

a) performing the method of claim 1 for at least one other pathway and

b) comparing the score of said pathway to the score(s) of said other pathway(s).

In one aspect of the invention, it is preferred that the pathways have the same characteristics. A characteristic of a pathway, as used herein, is defined as any quantitative property of the abstract interaction network described by the pathway. In a preferred embodiment, this property is of biological relevance or the scoring function is sensitive to

it. Suitable characteristics of pathways include the size, the length, the width and the diameter of pathways, as defined in [20].

In one embodiment of the invention, the scores of pathways are calculated from scores for individual molecules. Said scores for individual molecules are calculated from the experimental quantification of amounts of molecules.

In another embodiment, the scores for pathways are calculated directly from the experimental quantification of amounts of molecules without requiring calculation of scores for individual molecules.

In a further specialization of the latter embodiment, additional scores for individual molecules are calculated from the scores for pathways. These additional scores for individual molecules indicate a degree of relatedness of said molecules to said pathways. According to the present invention the pathways may be complete or partial. Suitable data for the method of the present invention comprise gene expression data and protein expression data. The pathways may be constructed from established biological knowledge and/or from hypotheses.

A suitable method for the representation and construction of pathways are Petri nets [29, 32] known to those skilled in the art. The method of the present invention is suitable to compare at least two different states of one biological system, e.g. before and after some kind of treatment or healthy and diseased cells, or the states of two different biological systems may be compared, e.g. cells from different organisms biological systems.

The method of the present invention is especially suitable

- for finding biologically realized pathways in a biological system,
- for identifying molecules that do not form part of the complete pathway corresponding to a given pathway, or
- for identifying molecules that form part of the complete pathway corresponding to a given pathway.

The method of the present invention is also specially suitable

- to identify pathways that are biologically realized or missing in a disease status, or
- to identify pathways that are biologically realized in only one, some, or all biological systems under investigation.

## Detailed description of the invention

The method of the present invention compiles the available facts on chemical reactions and regulations from the corresponding databases and represents these facts as interaction networks in the from of Petri nets [11, 29, 32]. Petri nets are well studied graph like concepts, which are accompanied with an extensive, well-established theory. Petri nets are especially well-suited for representing metabolic and regulatory relationships in a natural and straightforward way [31]. The available knowledge on molecular relationships can, with the help of Petri nets, be formulated in a uniform language and, additionally, it is made directly accessible to graph and simulation algorithms that are useful for the investigation of biochemical pathways.

Complete pathways according to the invention are consistent with user-defined constraints and define the range of possible or plausible pathways to connect molecular units in biological systems. Whether or not these pathways are indeed realized by a system under certain conditions or in certain states cannot be derived from current databases on reactions and interactions exploited for the pathway generation alone. The data needed to address these questions are increasingly becoming available by the above mentioned expression level measurements on large sets of genes.

In one embodiment, this invention describes a method, which exploits the expression data via a statistical scoring device and which allows to find realized pathways compatible with the measurements and, at the same time, allows for an evaluation of the raw expression data. Based on the background of the scored expression values for complete pathways, the procedure allows for identifying new genes correlated with the respective pathway, which could be worthwhile starting points for new pathways to be investigated or could indicate new relationships beyond current knowledge. Additionally, again based on the scored pathways and its constituents, the procedure indicates in quantitative detail which parts of the pathway are high- or low-scoring, respectively. The latter hints to problems in the experimental procedure or with the assumed relationships and indicates directions for further experiments and measurements.

For the evaluation of specific expression measurements the nets derived from the available databases can be complemented by specific expert knowledge on the problem domain and the experimental setup, on specific genes and gene products related to the experiment and/or disease state, on known interactions, both on the metabolic and regulatory side of relevant units, and on partial pathways known from other experiments or described in the literature. The system for enumerating pathways has explicit means to specify such additional knowledge in a unified way, that is also used for the automatic derivation of network information. Thus, expert knowledge on certain states to be investigated (e.g. cell types, diseases, etc.) can easily be phased into the process by globally specifying the validated or hypothesized knowledge as a user defined interaction network can easily be phased into the process by globally specifying the validated or hypothesized knowledge as a user defined interaction network.

Petri nets allow for the manual modification of generated or previously constructed networks in order to augment the network by specific individual knowledge of experts. This directly enables experts to add additional facts, to formulate hypotheses, and to specify contradicting alternatives. This information, in the form of an extended network, can be used to evaluate a whole range of experiments as described above. The different hypotheses or alternatives can be evaluated and thus allow to identify possible targets for interrupting or stimulating specific pathways.

The method of the present invention explicitly allows for feeding hypotheses or biological intuition or pharmaceutical ideas on potential targets into a method for target finding. The system is able to evaluate the proposed hypotheses together with the established knowledge against the new experimental evidence given by the expression level measurements. Alternative or contradicting hypotheses can be weighted against each other in the above context and, thus, the best alternative can be selected. Ideally, such a hypothesis is a complete pathway considered to be important for the biological system under investigation and providing hints for possible targets. Furthermore, an iterative process can be performed, which, based on previous hypotheses and the outcome of the corresponding expression experiments, allows to optimally design new experiments which further enhance the knowledge on the system and finally validates target candidates as far as is possible with this kind of experiments and analysis.

Due to the error rates of the current expression level measurements, methods that rely on the comparison of individual gene products are bound to be very unreliable themselves. This is a major drawback of the useful evaluation of expression data.

An important feature of the present invention is to combine the generation of possible and plausible pathways with the evaluation of expression data using a statistical score, which rates a complete or partial pathway with respect to the measured expression data. This score may be compared to the scope of all other possible pathways or to those of random pathways. The score combines evidence from a complete set of measurements, each of which might be quite unreliable. Thereby, the score relies on many measurements and their relative difference to a large set of other measurements. Additionally, the score evaluates complete biological units as compared to individual reactions and units.

There are many ways to phase information about the topology of the pathway and their semantics into the calculation of the individual scores and the scores for complete pathways. For example, the expression levels should, in most cases, be the more correlated, the closer the respective gene products are on the path. Furthermore, if additional knowledge on the function of gene products is available or can be derived from the network, this could be indicative of whether a significant change in expression level should be expected or not. This can be taken into account via the specific design of the scoring function.

Figure 1 discloses a scheme of the embodiments of the invention.

One embodiment of the invention is disclosed below

A: Pathway Generation as disclosed in further detail in [20] and [21] (incorporated by reference)

A1: Compile the available knowledge on biologically relevant reactions and interactions between gene products including hypotheses into a graph-like notation (Petri nets);

A2: Compute all biologically possible paths which are of interest in a specific pharmaceutical or biological context;

A3: Generate a set of random paths of similar characteristics (e.g. length, size and diameter). The result is a set of pathways, i.e. complete sets of genes.

B: Scoring Pathways

B1: Measure the expression levels with an appropriate experiment and measuring device and determine normalized differences between the states to be compared for each gene product;

B2: Compute the score for any possible pathway with respect to the statistical model, the actual expression level differences and the topology of the respective pathway;

B3: Compare the scores of the various pathways and select those with significant evidence as compared to the scores of random paths. The result is a set of scored pathways.

C: Evaluation of Expression Data

The most significant pathways indicate not only the desired subset of genes, which are of most interest in the evaluation of the current expression experiment data, but, additionally, the topology of the path specifies very detailed chains of reactions and interactions between molecular units. Therefore, those pathways are a good basis for understanding the differences between states in the context of the actual measurement and the current biological knowledge as available for the pathway generation of steps A1-A3.

This allows for:

C1: Design of further expression measurements or other experiments.

C2: Selection of target pathways and the detection of target gene products for the subsequent drug development process.

C3: Formulation of new hypotheses by human experts, which use the computed target pathways together with additional knowledge from the literature and/or other experiments to construct new Petri nets.

Figures

Figure 1: Overview of the method of the invention illustrating the general description of the invention.

Figure 2: Paths and Pathways: The figure shows two paths from source to sink of length 4 and 5, respectively. The pathway for a path contains the path and is closed, i.e. contains all non-ubiquitious substrates connected to the path. In this case, the pathways (the subnet indicated by the encircled region) of both paths from source to sink are the same.

Figure 3: Swissprot entry P39113 describing an activator protein for the transcription of two of the key enzymes in the gluconeogenesis pathway.

Figure 4: Petri net extending the Petri net of the gluconeogenesis (right side part) by the regulatory transition (left) defined by Swissprot entry P39113.

Figure 5: Overview of the glycolysis as computed from the unified Petri net derived from the metabolic databases KEGG, ENZYME, and BRENDA containing all pathways with a maximum width of 1 from D-Glucose to Pyruvate as computed with the enumeration algorithm described in [21]. The width has to be at least 2 in order to include the textbook-glycolysis in the set of valid pathways. Note that individual pathways cannot be distinguished in this form of illustration.

Figure 6: Pathways of the glycolysis as computed from the unified Petri net derived for the metabolic databases KEGG, ENZYME, and BRENDA containing all pathways with with a maximum width 2 as computed with the enumeration algorithm described in [21]. The width has to be at least 2 in order to include the textbook-glycolysis (thick lines) in the set of valid pathways. Note that individual pathways cannot be distinguished in this form of illustration.

Figure 7: Differential Metabolic Display (DMD) of the glycolysis for yeast and MG (Mycoplasma Genitalium) genomes containing all pathways of width 2 for all paths from starting reactant D-glucose to ending product pyruvate. This Figure contains all enzymes of Figure 6 for which sequence information is available. The thick edges indicate pathways present in both organisms (MG and yeast), thin black lines are found only in yeast pathways, but not in MG. No pathways are present in MG and not in yeast in this example. Dotted lines indicate enzymes known from other organisms than yeast or MG.

Figure 8: Histogram of the log-relative expression level (equation 1) distribution for time point 1 of the DeRisi measurement series [10] for all yeast genes.

Figure 9: Example pathway drawn from Figure 6. This path and the associated evaluation of the scoring function is discussed in detail in the main text. The path contains ten enzymes, the transitions of which are annotated with the associated EC numbers and the yeast ORF identifiers.

Figure 10: Graphical illustration of the data shown in Table 1. Each line corresponds to the expression of one of the genes involved in the example glycolysis pathway during the seven time points.

Figure 11: Histogram of the pathway scores calculated according to Equation 10 for all possible assignments of the glycolysis pathway reactions to yeast ORFs.

Figure 12: Histogram of the pathway scores calculated according to Equation 10 for 10000 random pathways. Since the pathway-model used for this example does not account for the graph structure of the pathways, but is sensitive to the size of the pathways, the random pathways are subsets of all genes of the same size (i.e. 10 genes) as the

glycolysis pathway.

Figure 13: Histogram of the TCA cycle scores calculated similarly as in Figure 11.

Figure 14: Histogram of scores of random pathways of the same size as the TCA cycle pathway, analogous to Figure 12.

Figure 15: Graphical illustration of the time series of expression levels of a TCA pathway (i.e. assignment of ORFs to the TCA cycle transitions). Each line corresponds to the expression of one of the genes involved in the example pathway during the seven time points.

Table 1: Expression data as used for the score calculation of a pathway consisting of the ten genes shown. The data are taken from [10]. There is one row of data for each gene included in the pathway as identified in the first column. Each data column corresponds to a time point from t1 to t7. The data shown are the values $r_{t,g}$ as defined in Equation 1: the logarithms of the ratios of the measured gene expression at the indicated time point to the expression at the base time point t0.

Table 2: Mean values for the simple pathway-models as described in Equation 2, calculated from the data shown in Table 1. In order to avoid the influence of self-correlation on the scores of genes included in the pathway, each gene is removed from the pathway before the particular pathway-model is built that is used for the computation of the score of the respective gene.

Table 3: Empirical standard deviations for pathway-models according to Equation 3 similar to Table 2.

Table 4: Mean values and empirical standard deviations for the null-models. The parameters of a null-model for each time point t1 to t7 is calculated from the data described in [10] using Equations 4 and 5.

Table 5: The scores for the genes included in the pathway for the different time points, computed according to Equation 8. The values in the last column, titled *average,* correspond to the gene scores according to Equation 9.

## Description of Methods

### *Pathways*

In order to facilitate a system (e.g. cell-, tissue-, organism-, or species-) wide, holistic evaluation of sequence and expression data we compiled the available data of metabolic databases into Petri nets. Petri nets are graph-like structures that lend themselves naturally to representing all kinds of relations and interconnections of distributed interacting entities (substrates, proteins) in a metabolic/regulatory network. In the context of this invention, Petri nets derived from available databases and additional expert input are used to provide the biological background knowledge for the analysis of expression data, especially in order

- to merge all available databases to integrate the stored biochemical facts and to remove inconsistencies,
- to generate (if desired, cell-type specifically) all putative pathways that can be subjected to our new method to evaluate pathways by expression data,
- to define and analyse interaction networks by their underlying structure of paths and pathways,
- to compare genomic and expression information with knowledge about interaction networks and
- to define a notion of Differential Metabolic Display (DMD) that allows to compare specific systems, i.e. organisms, developmental or disease states, by comparison of the individual Petri nets.

The main sources of information about biochemical pathways are databases like BRENDA [19], ENZYME [2], KEGG/LENZYME [25], MPW [34], WIT [28], EcoCyc [19], and HincCyc [18] containing textual descriptions of reactions. Regulatory relations are inferred from sequence database annotations (i.e. Swissprot [3], Prosite [4]) or from literature abstracts (Medline http://www.ncbi.nlm.nih.gov/entrez/).

The compilation process of the different databases used and the removal of mistakes and inconsistencies and the unification of the database format is described in detail in [21].

The main purpose of the compiled Petri nets for pathway databases is the systematic generation of paths and pathways in such nets to facilitate the analysis of differences between certain environmental states, between different organisms (genomes) and between different cell types of one organism. Petri nets with their underlying semantics [21] (the so called "firing rule") and additional user defined and biologically motivated restrictions [21] enable to drastically reduce the number of valid paths leading from a set of educts to a set of products.

Based on such restricted valid paths, a concept of *pathways* is defined: Given a Petri net, a *pathway* associated with a path is a partial net that contains the path and is *closed. Closed paths* account for the availability of educts and

take care of the consumption of intermediate products. User definable sets of ubiquitious educts and products then allow to determine pathways of different extent. Additionally, notions of length, diameter, area, and width of pathways are introduced to enable the generation and analysis of pathways with specific, pre-defined properties (Figure 2). The length of a pathway is the length of the longest path from source to sink contained in the pathway, its diameter is defined as the length of its longest path, its area as the number of transitions (enzymes/genes) in the pathway, and its width as the size of its maximal ST-cut.

Search and generation algorithms for paths and pathways of Petri nets which allow for user defined constraints using these parameters in order to restrict the valid paths for further analyses have been developed [21]. The resulting pathways are optimally suited for being evaluated with the method of the invention, as, by definition, they represent biologically meaningful units. The enumeration process is described in detail in [21]. As a result from this method, the set of all known biologically meaningful pathways connecting predefined sources and sinks is obtained.

On the basis of the complete set of restricted pathways, it is possible to systematically compare different networks, i.e. different developmental or disease states of different organisms. For this purpose so called Differential Metabolic Displays (DMDs) have been introduced [20]: system specific subnets are extracted from the unified Petri nets and the respective intersection and difference sets for different systems are determined. A DMD can be represented as a Petri net containing pathways, colored according to the above system-specific sets to simultaneously exhibit shared, missing, and specific pathways for each system under consideration.

DMDs allow to display significant differences, to identify gaps in specific pathways, and to enable the evaluation of expression data by making predictions for proteins of unknown function and to propose the existence and/or absence of specific proteins or protein functions in certain systems.

The information in currently available databases can be enriched by functional knowledge acquired by human experts from academic or industrial research groups. This exploits a type of functional information quite different from the function (i.e. biochemical reactions) stored in metabolic databases (Figure 3) and tries to make them available in a uniform Petri net setup (Figure 4). Thus, the relationships in the Petri nets can be extended significantly with specific relations or functional specifications relevant for the special interest of the human expert.

As an example for adding expert knowledge is given: Entry no P39113 of the Swissprot database (Figure 3) specifies that certain proteins of the glycolysis are regulated by the transcriptional activator CAT8. This information results in the construction of the extended net as shown in Figure 4.

Example

As an example, the paths and pathways of the glycolysis are considered.

In contrast to the simplified textbook view of metabolic pathways, the number of potential, unrestricted paths connecting two proteins or metabolites in the network appears to be very large (some 500.000 paths of length at most nine from glucose to pyruvate, not shown). In general, this prohibits the systematic analysis of all potential paths. The application of the Petri net firing rule (step 1 and 2) reduces the number of paths to (still) about 80.000 paths involving some 800 enzymes (not shown). Exploiting additional pathway constraints (restricting the cut-width to 2 and 1) in steps 3 and 4 results in 541 and 170 pathways, respectively (Figures 5 and 6).

Nets restricted to specific genomes (see Figures 5, 6 and 7), after having mapped the sequence data, can be used to find and exhibit detours and gaps in organism-specific metabolic pathways and to propose protein functions to be searched for in genomic data to complete apparently disrupted pathways. E.g., for the yeast and *Mycoplasma Genitalium* (MG) genomes 550 pathways with 225 reactions are in the current metabolic databases. Out of these, sequence information of 185 of the 225 enzymes could be assigned. The light grey lines indicate all edges contained in the remaining 140 pathways consisting of enzymes with known sequences represented in the current sequence databases. The thick edges indicate paths contained in pathways present in both organisms (MG and yeast), thin black lines are found only in yeast pathways but not in MG, dotted lines would have indicated paths in MG not present in yeast (none in this example).

*Calculation of statistical scores*

This section describes the definition of scores for genes and pathways based on expression level measurements and the combination of pathway generation and statistical pathway evaluation with the goal to select a set of most interesting pathways for a given expression measurement.

To be specific, the basic problem is:

- Given the **Input:**
    - Gene expression measurements of different states.

- Putative pathways that could or could not be realized in these states.

- Answer the **Questions:**
  - Which pathways are in fact realized in the cells?
  - Which genes do not have much support by the current measurements to belong to the pathway ?
  - Which genes not included in the putative pathways are likely to be related to the pathways?

- By producing the **Output:**
  - For each gene, both included and not included in the pathway, a score how well it fits the putative pathway according to the expression data.
  - For the putative pathway as a whole, a statistical score how probable it is that the pathway is realized in the cell type under investigation.

The basic idea is to rate the genes involved in a putative pathway, as well as the remaining genes, with respect to this pathway according to the behaviour of the expression of all genes. In order to do so, two statistical models are constructed, one model of the expression of the genes included in the pathway (pathway-model), and a second model of the remaining genes (null-model or background-model). Each gene, whether included in the putative pathway or not, can be compared to both models, and a score can be computed that reflects how much better it fits the pathway-model than the null-model and vice versa. If this is desired, one of the models can be chosen to be uniform, i.e. assigns equal probability to every gene, disregarding the observed expression behavior. This is amounts to omitting the corresponding model.

According to this idea, not only a given pathway itself can be rated, but in addition each gene from the pathway individually as well as each remaining gene can be rated with respect to expression correlation to the pathway. This offers the opportunity to augment the knowledge about the pathways by identifying, on one hand, not similarly expressed genes within the pathway and, on the other hand, similarly expressed genes that are not yet linked to the pathway. This works the better, the stronger the gene expression experiments involves the regulation of the pathway under investigation, and the better the models of expression behavior reflect the biological reality. Thus, the models should be calibrated using available measurement data.

<u>Definition of the Scoring System</u>

Thus, for a concrete application of this principle to a putative pathway, crucial technical choices have to be made regarding two points:

First, a number of gene expression assessment experiments have to be selected from the data sets already available, or have to be newly designed and performed. Additionally, relative weights can be assigned to the different experiments or, in the case of time series measurements, even to individual time points.

Second, a gene pathway scoring (GPS) function has to be defined, that assigns to each gene a score that reflects its correlation to the genes belonging to the pathway as opposed to the remaining genes. This is closely tied to the definition of the mathematical models for these two sets of genes. In the case of probabilistic models, the log-odds ratio of the probabilities (as shown in the example below, Equation 8) is a natural choice for the scoring function. A good scoring function has to reflect expression behavior resulting from the different types of possible biological connections. With respect to expression time series features like proportionality (common regulation), reciprocal proportionality (synchronized regulation), time delayed correlation (one side regulates the other side) and the like can be exploited in order to capture complex regulatory relations. On the side of the pathways, the graph structure of the involved genes as given by the pathway can be taken into account, for example by giving more weight to the influence of the correlation of genes that are close to each other in terms of the shortest path between them. However, in the example below it is demonstrated that a comparatively simple function that corresponds to a simple pathway-model already leads to reasonable results.

Depending on the definition of the scoring function, the score distribution may depend on the characteristics of the pathways scored, most importantly the size. This hampers the comparison of scores of pathways of different characteristics. Statistical scores, called *p-values* (probability estimates) or E-*values* (expectation values), that remedy analogous problems in the field of sequence comparison have been an important pre-requisite for the success of programs like BLAST and FASTA for that application. These scores, in addition to increasing the reliability of decisions

based on them as opposed to other scores, have an intuitive interpretation as probabilities or expectation values of erroneous decisions, and can be used to guide the trade-off between sensitivity and specificity. The computation of similar p-values for pathways is preferred, for example by the following procedure: for each putative pathway under investigation, a large number of random pathways with the same characteristics are generated and scored. Then, the p-value of the pathway under investigation is taken as the fraction of random pathways that achieve the same score or a greater score. This p-value is an estimate of the fraction of false positives to be expected when assuming that the pathways under investigation are realized in the specific cell states represented by the current measurement.

Rating and adapting putative pathways by gene expression measurements

1. For both the pathway *p* and the remaining genes $\bar{p}$, derive from the expression measurements a probabilistic model that describes the properties of the gene expression behavior of the respective set of genes.

2. For each gene *g*, both from *p* and from $\bar{p}$, compute a score $score_p(g)$ that reflects how well its expression behavior fits the pathway-model in contrast to the null-model. The scores of the genes not included in the pathway can be used to identify genes that are possibly related to the pathway.

3. From the scores of the individual genes included in the pathway, compute an overall score $score_p$ for the pathway.

4. Compute a *p-value,* i.e. an estimate of the statistical significance of the pathway score. This can be, for example, the fraction of random scores that exceed the score of the given pathway. Appropriate random scores can be computed by applying steps 1-3 of this procedure to randomly created pathways sharing the characteristics (size, length, width etc.) of the pathway under investigation.

5. Based on the *p-value,* accept or reject the hypothesis that the pathway is realized in the type of cells that were subject to the expression measurement.

Example realization of the scoring function

In order to show the efficacy of our procedure, we investigate the glycolysis pathway in *saccharomyces cerevisiae* (yeast). We make use of the gene expression time series measured by DeRisi et al. [10] that is publicly available (http://cmgm.standford.edu/pbrown/explore/index.html). For each known yeast gene g there are measurements $I_{t,g}$ represented as real numbers (see table below) of the expression level for a set of different time points t. This time series is optimally suited for the rating of the glycolysis pathway as the time points correspond to decreasing concentrations of glucose available to the yeast and a regulation of the glucose processing glycolysis pathway can be expected. In fact, the data measured confirm this expectation [10] as demonstrated by a manual analysis of the data by the original authors.

Investigation of the distribution of the relative changes of gene expression for the different time points with respect to the base level corresponding to time point $t_0$. For each time point t, we take the logarithm of the expression change ratio,

$$\log \frac{I_{t,g}}{I_{t0,g}},$$

for each gene *g*. In the following, let $r_{t,g}$ denote the log-relative expression levels

$$r_{t,g} = \log \frac{I_{t,g}}{I_{t0,g}} \qquad (1)$$

This leads to a distribution of values that is symmetrical with regard to up- and down-regulation. While the resulting distributions are not necessarily normal, they share some important characteristics with normal distributions. They are sigmoid, and the density functions are unimodal and almost symmetrical (see Figure 8). Thus, they can be approximated by normal distributions without making a qualitative error. It is taken advantage of this observation in order to construct the models used in the scoring function.

For each time point, two sets of expression values are collected, corresponding to the set of genes involved in the pathway (denoted *p*) and the set of remaining genes (called $\bar{p}$), respectively. For both sets, the log ratios described above are fitted to normal distributions by simply taking the mean rand the empirical standard deviation s of the sets. As an example, equations 2 and 3 show, how this can be done for the set of genes *p* belonging to the path for time point *t*:

$$\bar{r}_{t,p} = \frac{1}{|p|}\sum_{g \in p} r_{t,g} \quad (2)$$

$$s_{t,p} = \sqrt{\frac{1}{|p|-1}\sum_{g \in p}(r_{t,g} - \bar{r}_{t,p})^2} \quad (3)$$

Analogously, the mean $\bar{r}_{\bar{p},t}$ and the standard deviation $s_{\bar{p},t}$ can be computed for the set of genes $\bar{p}$ not belonging to the path for each time point *t*:

$$\bar{r}_{t,\bar{p}} = \frac{1}{|\bar{p}|}\sum_{g \in \bar{p}} r_{t,g} \quad (4)$$

$$s_{t,\bar{p}} = \sqrt{\frac{1}{|\bar{p}|-1}\sum_{g \in \bar{p}}(r_{t,g} - \bar{r}_{t,\bar{p}})^2} \quad (5)$$

For each gene g, a score is computed that reflects how well it fits the path. For this purpose, the gene is removed from the set it is assumed to belong to (either *p* or $\bar{p}$), resulting in the sets *p*-{*g*}and $\bar{p}$-{*g*}. This is most important when the size of the set is small and the presence of the gene has a considerable effect on the estimated distribution. First, an estimation *P* for the probability of the gene g to belong to the path *p*-{*g*} or to the set of remaining genes $\bar{p}$-[*g*}, respectively, is approximated using the normal distribution Φ. This is done for each time point t individually.

$$P_{t,p}(g \mid p - \{g\}) := 2 * \Phi\left(-\left|\frac{l_{t,g} - \bar{r}_{t,p-\{g\}}}{s_{t,p-\{g\}}}\right|\right) \quad (6)$$

$$P_{t,p}(g \mid \bar{p} - \{g\}) := 2 * \Phi\left(-\left|\frac{l_{t,g} - \bar{r}_{t,\bar{p}-\{g\}}}{s_{t,\bar{p}-\{g\}}}\right|\right) \quad (7)$$

This definition of the probabilities rests on the assumption that it is the more probable that a set forms a pathway that is realized in the investigated cell types, the more correlated (e.g. proportional) the expression of this set of genes is. This is especially true for the pathway model, whereas the null-model is reasonably justified by the empirical log-ratio distribution observed above. In general, synchronization of expression can be more sophisticated than mere

proportionality, and accordingly more elaborate models (especially for the pathway) can be devised as mentioned above. Still, these definitions of probabilities lead to a scoring function that can be useful as shown below.

The score of g is calculated as the log-odds score of the approximated probabilities of the gene under investigation to belong to the path $p$-{$g$}or to the set of remaining genes $\bar{p}$-{$g$} for each time point $t$:

$$score_{t,p}(g) := \log \frac{P_{t,p}(g|p\text{-}\{g\})}{P_{t,p}(g|\bar{p}\text{-}\{g\})} \qquad (8)$$

The over-all score for the gene $g$ with respect to the complete time series or set of states/measurements can, for example, be computed as the simple average over the set $T$ of all time points $t$:

$$score_p(g) := \frac{1}{|T|}\sum_{t\in T} score_{t,p}(g) \qquad (9)$$

A score for the complete pathway can be computed as the average over the scores of the genes included in the pathway:

$$score_p := \frac{1}{|p|}\sum_{g\in p} score_p(g) \qquad (10)$$

Using the pathway generation method described above, all pathways from glucose to pyruvate were generated. These pathways are characterized by the types of reactions needed to build pyruvate from glucose in a number of steps and by the graph structure that these reactions impose on the enzymes and the intermediate substrates. With appropriate constraints this results in the pathways shown in Figures 5 and 6.

For this example, we select one of the generated paths, the pathway marked as bold in Figure 6.

This pathway, containing EC numbers (i.e. enzymatic functions) as transitions, generically represents a number of different pathways on the basis of individual genomic yeast open reading frames (ORFs) (corresponding to genes the product of which can perform the required reaction) shown in Figure 9.

Thus, the EC-pathway induces a set of gene-pathways. By construction, the size of all the pathways, i.e. the involved number of the reactions respectively the proteins, is constant - in this case, ten.

In Table 1, the identifiers (IDs) of the yeast open reading frames corresponding to one possible assignment of proteins to the pathway are shown, together with the logarithms of the ratios of expression values measured at different time points with respect to the base time point to in the diauxic shift expression assessment [10]. The data from the Table are visualized as time curves in Figure 10.

For this pathway, scores according to the Equations 1 to 10 are calculated as an example case. From the values in the table, sufficient statistics for the pathway-models according to Equations 2 and 3 are calculated. Since the pathway consists of only ten genes, each gene has a non-neglectable influence on the pathway-model. Therefore, a different pathway model for each gene included in the pathway was calculated, as needed for Equation 8. This leads to the mean values and empirical standard deviations shown in Tables 2 and 3.

Using Equations 4 and 5, sufficient statistics of the null-models can be computed. Therefore, the expression values of the genes not included in the pathway, i.e. the remaining 6249 genes of the known yeast genes investigated in [10] are needed (this expression data is not shown here). Since each single gene has a neglectable influence on the statistics of this set, we simplify this example by approximating the correct null-models for $\bar{p}$-{$g$} by the null-models for $p$. The resulting values for mean and standard deviation are shown in Table 4.

Applying Equations 6 to 8, the scores for the genes included in the pathway are revealed [shown in Table 5]. Accordingly, this pathway is assigned a $score_p$ of 0. 736.

Application of the same procedure to the set of all pathways in terms of sets of yeast ORFs that can be assigned to the reactions in the generated glycolysis pathway leads to the distribution of scores shown in Figure 11.

In contrast, the analogous computation of scores for 10000 randomly chosen ORF sets of size 10, that form a sufficient random model with respect to the used scoring function, leads to a distribution of scores as shown in Figure

12.

It is easy to see that the scores of the glycolysis pathway lie well above the scores expected from random paths. This confirms the hypothesis that the glycolysis pathway is realized in the investigated states of yeast.

This result can not be achieved using clustering methods, because the genes encoding the involved enzymes are not similarly regulated (at least this does not manifest in the current measurements), as was already observed in [10] and can be seen in Figure 10.

The method of the present invention, even with the simple example statistical model as described in the previous section, can recognize realized pathways with heterogeneous regulation.

For the example glycolysis pathway defined above, a p-value of *0.0009* can be derived from the random score distribution by determining the fraction of random pathways that score equal or better than $score_p$. This is a very good result and is - given the data shown in Figure 10 - very hard to match with clustering based methods.

Another example illustrates this point: For the textbook tricarboxylic acid (TCA) cycle, a supposedly easier example, our method performs even better. Excellent scores as shown in Figure 13 are achieved. Here, even the lowest TCA pathway score is better than the highest score of 10000 random pathways with equal length (shown in Figure 14), whereas, again, the accompanying expression level time series (Figure 15) do not cluster together easily in non-trivial discriminating clusterings.

Experiment Design

The above methods for deriving and representing networks, the generation of pathways with specific characteristics and for the subsequent calculation of scores can be applied for the subsequent calculation of scores can be applied for improving the design of further experiments and experimental measurements, by performing the following steps:

- measuring the new data in the expression experiment in order to provide the enhanced discrimination between the various hypotheses to be tested
- designing the experiments based on the hypotheses fed into the system, e.g. formulated in augmented Petri nets
- designing the experiments to account for the type of statistical score used for the subsequent evaluation
- planning the experimental setups such that the already measured data is used to avoid unnecessary experimental duplication
- placing normalization measurements at crucial points in the experimental setup to allow for optimal usage of precious material, i.e. patient tissue of certain disease states
- connecting measurements made on readily available in vitro material with measurements on in vivo material for the evaluation
- designing additional experiments such that ambiguities in the scoring based on the previous experiments alone are removed and such that the resulting statistical score is optimized
- designing the experiments such that the DNA chips or other experimental equipment is used efficiently, i.e. the number of consumed resources is minimized for the information obtained.

[1]
*The Chipping Forecast,* volume 21. Nature, January 1999.

[2]
Bairoch A. The ENZYME data bank in 1999. *Nucleic Acids Res,* 27(1):310-311, 1999.

[3]
A. Bairoch and B. Boeckmann. The SwissProt protein sequence data bank, *Nucleic Acids Res* 1992.

[4]
A. Bairoch, P. Bucher, and K. Hofmann. The PROSITE database, its status in 1997. *Nucleic Acids Res* 25(1): 217-221, 1997.

[5]
Michael P. S. Brown, William Noble Grundy, David Lin, Nello Cristianini, Charles Walsh Sugnet, Terrence S. Furey, Manuel Ares, and David Haussler. Knowledge-based analysis of microarray gene expression data by using support vector machines. *Proceedings of the National Academy of Sciences of the USA,* 97(1):262-267, January 2000.

[6]
Daniel B. Carr, Roland Somogyi, and George Michaels. Templates for Looking at Gene Expression Clustering. *Statistical Computing & Statistical Graphics Newsletter,* 8(1):20-29, April 1997.

[7]
Mark Chee, Robert Yang, Ear Hubbell, Anthony Berno, X.C. Huang and David Stern, Jim Winkler, David Lockhart, Macdonald Morris, and Stephen A. Fodor. Accessing genetic information with high-density DNA arrays. 274(25 Oct):610-614, 1996.

[8]
S. Chu, J. DeRisi, M. Eisen, J. Mulholland, D. Botstein, P. O. Brown, and I. Herskowitz. The Transcriptional Program of Sporulation in Budding Yeast. *Science,* 282:699-705, October 1998.

[9]
Schomburg D., Salzmann D., and Stephan D. *Enzyme Handbook,* Classes 1-6. Springer, 1990-1995.

[10]
Joseph L. DeRisi, Vishwanath R. Iyer, and Patrick O. Brown. Exploring the metabolic and genetic control of gene expression on a genomic scale. *Science,* 278:680-685, October 1997.

[11]
S. Drees, D. Gomm, H. Pünnecke, W. Reisig, and R. Walter. *Bibliography of Petri Nets 1988,* volume 315. GMD Arbeitspapiere, 146pp edition, 1982.

[12]
Michael B. Eisen, Paul T. Spellman, Patrick O. Brown, and David Botstein. Cluster analysis and display of genome-wide expression patterns. *Proceedings of the National Academy of Sciences of the USA,* 95(25):14863-14868, December 1998.

[13]
M. Fellenberg and H. Werner Mewes. Interpreting Clusters of Gene Expression Profiles in Terms of Metabolic Pathways. In *Proceedings of the German Conference on Bioinformatics '99,* 1999. Poster.

[14]
David Gerhold, Thomas Rushmore, and C. Thomas Caskey. DNA chips: promising toys have become powerful tools. *Trends in Biochemical Sciences,* 24(281):168-173, May 1999.

[15]
R. Heller, M. Schena, A. Chai, D. Shalon, T. Bedilion, J. Gilmore, D. Woolley, and R. Davis. Discovery and analysis of inflammatory disease-related genes using cDNA microarrays. 94:2150-2155, 1997.

[16]
Renu A. Heller, Mark Schena, Andrew Chai, Dari Shalon, Tod Bedilion, James Gilmore, David E. Woolley, and Ronald W. Davis. Discovery and analysis of inflammatory disease-related genes using cDNA microarrays. *Proceedings of the National Academy of Sciences of the USA,* 94(6):2150-2155, March 1997.

[17]
L. Hunter, D. Searls, and J. Shavlik, editors. Petri Net representation in metabolic pathways. NLM, Bethesda, MD, AAAI Press, 1993.

[18]
P.D. Karp, C. Ouzounis, and S. Paley. Hinccyc: A knowledge base of the complete genome and metabolic pathways of h. influenzae. *In Proceedings of the ISMB'96 conference,* pages 116-129, 1996.

[19]
Peter D. Karp, Monica Riley, Suzanne M. Paley, Alida Pellegrini-Toole, and Markus Krummenacker. Eco Cyc: Encyclopedia of escherichia coli genes and metabolism.
*NAR,* 27(1):55-58, 1999.

[20]
R. Küffner, R. Zimmer, and T. Lengauer. Pathway analysis in metabolic databases via differential metabolic display (DMD). In *GCB99 conference proceedings,* pages 141-147, 1999.

[21]
R. Küffner, R. Zimmer, and T. Lengauer. Pathway analysis in metabolic databases via differential metabolic display (DMD). Submitted to Bioinformatics, 1999.

[22]
Lockhart et al. Expression Monitoring by Hybridization to High-Density Oligonucleotide Arrays. *Nature Biotechnology,* 14:1675-1680, December 1996.

[23]
Andrew Marshall and John Hodgson. DNA chips: An array of possibilities. *Nature Biotechnology,* 16:27-31, 1998.

[24]
G.S. Michaels, D.B. Carr, M. Askenazi, S. Fuhrman, X. Wen, and R. Somogyi. Cluster Analysis and Data Visualization of Large-Scale Gene Expression Data. In *Proceedings of the Pacific Symposium on Biocomputing '98,* volume 3, pages 42-53, 1998.

[25]
H. Ogata, S. Goto, K. Sato, W. Fujibuchi, H. Bono, and M. Kanehisa. KEGG: Kyoto encyclopedia of genes and genomes. *Nucleic Acids Res.,* 27:29-34, 1999.

[26]
K. Okubo, N. Hori, R. Matoba, T. Niiyama, et al. Large scale cDNA sequencing for analysis of quantitative and qualitative aspects of gene expression. *Nature Genetics,* 2:173-179, 1992.

[27]
Kousaku Okubo and Kenichi Matsubara. Complementary DNA sequence (EST) collections and the expression information of the human genome.
*FEBS Letters,* 403(3):225-229, February 1997.

[28]
R. Overbeek, N. Larsen, W. Smith, N. Maltsev, and E. Selkov. Representation of function: the next step. *Gene,* 191(1):1-9, 1997.

[29]
C.A. Petri. Kommunikation mit Automaten. *Schriften des Instituts für Instrumentelle Mathematik,* Bonn, 1962.

[30]
Graham Ramsay. DNA chips: State-of-the art. *Nature Biotechnology,* 16:40-44, January 1998.

[31]
V.N. Reddy, M.N. Liebman, and M.L. Mavrovouniotis. Qualitative analysis of biochemical reaction systems. *Comput. Biol. Med.,* 26(1):9-24, 1996.

[32]
W. Reisig. Petrinetze Eine Einführung. Springer verlag, 1982.

[33]
M. Schena, D. Shalon, R. Davis, and P.O. Brown. Quantitative monitoring of gene expression patterns with a complmentary DNA microarray. *Science,* 270(20 Oct):467-470, 199.

[34]
E.J. Selkov, Y. Grechkin, N. Mikhailova, and E. Selkov. MPW: The metabolics pathway database. *Nucleic Acids Res,* 26(1):43-45, 1998.

[35]
P. Spellman, G. Sherlock, M. Zhang, V. Iyer, K. Anders, M. Eisen, P. Brown, D. Botstein, and B. Futcher. Comprehensive Identification of Cell Cycle-regulated Genes of the Yeast Saccharomyces cerevisiae by Microarray Hybridization. *Molecular Biology of the Cell,* 9(12):3273-3297, December 1998.

[36]
Pablo Tamayo, Donna Slonim, Jill Mesirov, Qing Zhu, Sutisak Kitareewan, Ethan Dmitrovsky, Eric S. Lander, and Todd R. Golub. Interpreting patterns of gene expression with self-organizing maps: Methods and application to hematopoietic differentiation. *Proceedings of the National Academy of Sciences of the USA,* 96:2907-2912, 1999.

## Claims

**1.** A method for evaluating states of biological systems comprising the steps of

a) constructing a pathway comprising at least two molecules and their interaction network,

b) measuring expression data with an appropriate experiment and measuring device and

c) calculating a score for said pathway based on said experimental quantification of the amounts of molecules in said system, said score indicating an intensity of realization of said pathway in said state of said biological system.

**2.** The method of claim 1, followed by an estimation of the significance of said score of said pathway by the steps

a) performing the method of claim 1 for at least one other pathway and

b) comparing the score of said pathway to the score(s) of said other pathway(s).

**3.** The method of claim 2, wherein the pathways have the same characteristics, e.g. the area, width and/or length.

**4.** The method of claims 1 to 3, wherein

a) said scores for pathways are calculated from scores for individual molecules and

b) said scores for individual molecules are calculated from said experimental quantification of amounts of molecules.

**5.** The method of claims 1 to 3, wherein said scores for pathways are calculated from said experimental quantification of amounts of molecules without requiring the calculation of scores for individual molecules.

**6.** The method of claim 5, wherein additional scores for individual molecules are calculated from said scores for pathways, the scores for individual molecules indicating the degree of relatedness of said molecules to said pathways, wherein additional scores for individual molecules are calculated directly from said experimental quantification of amounts of molecules independently of said scores for pathways.

**7.** The method of claims 1 to 6, wherein the pathways are complete or the pathways are partial.

**8.** The method of claims 1 to 7, wherein the experimental quantification is based on gene expression data or wherein the experimental quantification is based on protein expression data.

**9.** The method of claims 1 to 7, wherein the molecules are selected from the group consisting of DNA molecules, RNA molecules, proteins, pre-proteins, oligo-peptides, organic molecules, or an-organic metabolites.

**10.** The method of claims 1 to 9, wherein the pathways are constructed from established biological knowledge and/or from hypotheses.

**11.** The method of claims 1 to 10, wherein Petri nets are used for the construction of the pathways.

**12.** The method of claims 1 to 11, wherein at least two states of one biological system or the states of at least two biological systems are compared.

**13.** The method of claims 1 to 12

- to identify pathways that are biologically realized in only one, some, or all biological systems under investigation.

- to identify pathways that are biologically realized or missing in a disease states,

- for identifying molecules that do not form part of the complete pathway corresponding to a given pathway, or

- for identifying molecules that form part of the complete pathway corresponding to a given pathway.

**14.** A method as defined in claims 1 to 13, taking into account the type of statistical score used for the subsequent evaluation according to claims 1 to 13, applied for enhancing and planning the design of experiments by

- planning the experimental setups such that the already measured data is used to avoid unnecessary experimental duplication and such that experimental equipment is used efficiently,

- placing normalization measurements at crucial points in the experimental setup to allow for efficient usage of precious material, i.e. patient tissue of certain disease states, by connecting measurements made with readily available in vitro material with measurements on in vivo material for the evaluation, or

- designing additional experiments such that ambiguities in the scoring based on the previous experiments alone are resolved and such that the resulting statistical score based also on the additional measurements is optimized and discriminating between specific pathways.

**15.** An iterative method in particular according to the claims 1 to 14 for intertwining the hypotheses formulation and experiment design, comprising the steps of

- selecting the most plausible pathways according to the current experimental data with the methods of claims 1 to 14 claimed above,

- modifying and enhancing the interesting pathways based on this analysis with new formalized hypotheses,

- deriving new experimental setups, which discriminate between alternative and/or contradicting hypotheses

- iterating these steps until enough information on potential target candidates has been assembled to proceed to subsequent steps of target validation and drug development or the network cannot be reliably further extended in step 2 above.

Figure1:

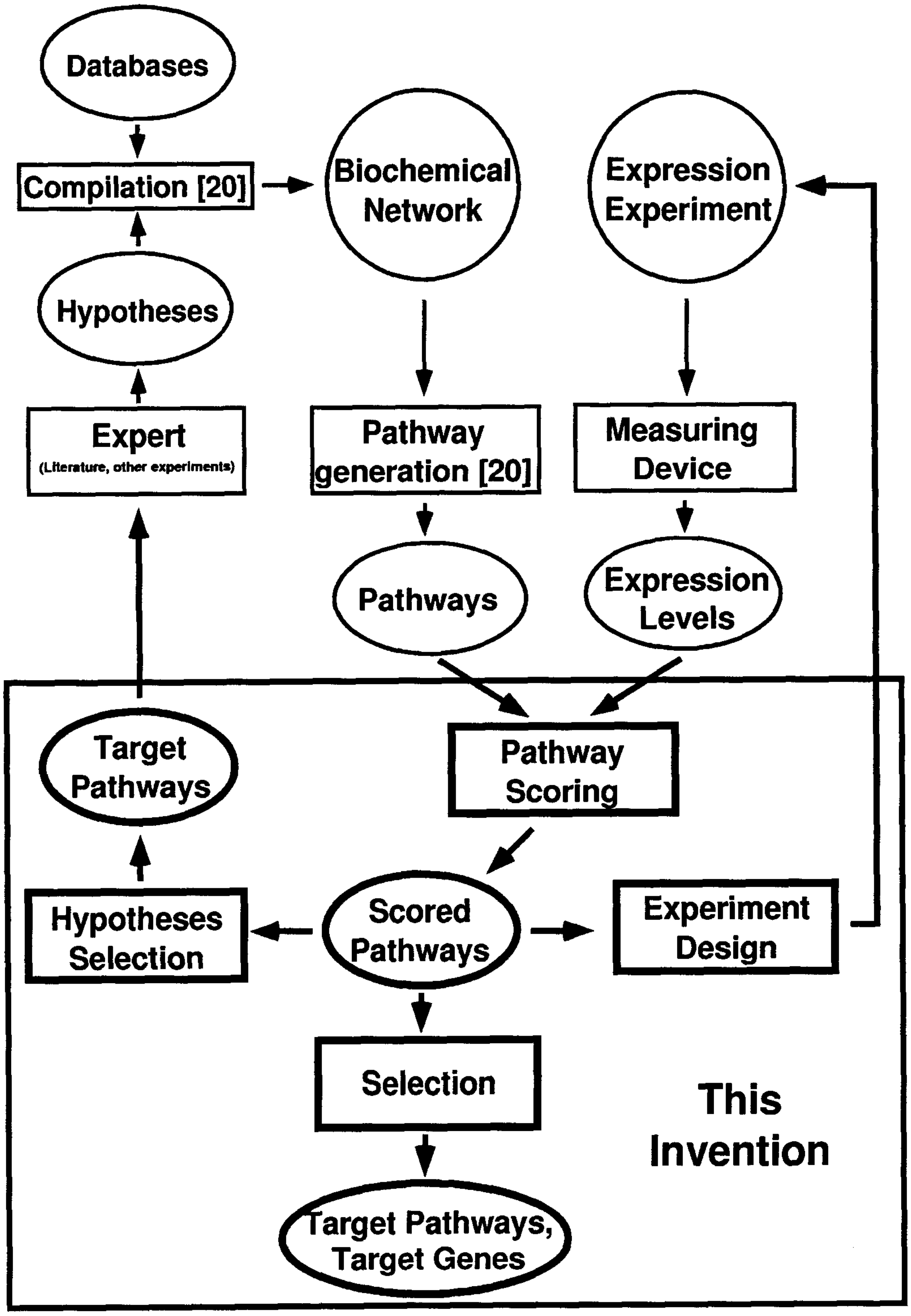
Databases
Compilation [20]
Biochemical Network
Expression Experiment
Hypotheses
Expert
(Literature, other experiments)
Pathway generation [20]
Measuring Device
Pathways
Expression Levels
Target Pathways
Pathway Scoring
Hypotheses Selection
Scored Pathways
Experiment Design
Selection
This Invention
Target Pathways, Target Genes

Figure 2:

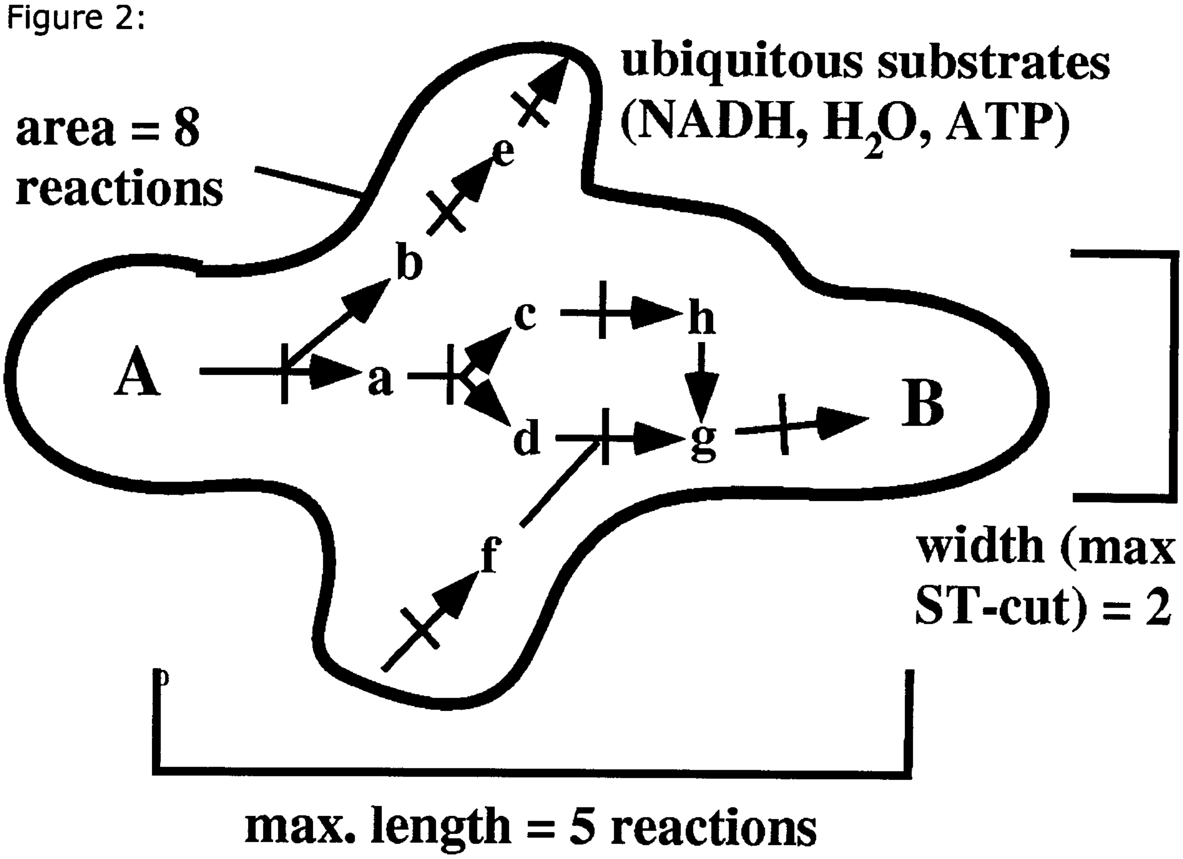
ubiquitous substrates (NADH, H2O, ATP)
area = 8 reactions
A
a
b
c
d
e
f
g
h
B
width (max ST-cut) = 2
max. length = 5 reactions

Figure 3:

```
ID   CAT8_YEAST     STANDARD;      PRT;  1433 AA.
AC   P39113;
DE   REGULATORY PROTEIN CAT8.
GN   CAT8 OR MSP8 OR YMR280C OR YM8021.06C.
OS   SACCHAROMYCES CEREVISIAE (BAKER'S YEAST).
OC   EUKARYOTA; FUNGI; ASCOMYCOTINA; HEMIASCOMYCETES.
...
CC   -!- FUNCTION: ACTIVATOR OF THE GLUCONEOGENIC
         ENZYMES FBP1 AND PCK1 GENES.
CC   -!- SUBCELLULAR LOCATION: NUCLEAR.
CC   -!- PTM: COULD BE THE TARGET OF THE SNF1/CAT1 -
         SNF4/CAT3 KINASE COMPLEX.
CC   -!- SIMILARITY: CONTAINS A ZN(2)-CYS(6), FUNGAL-
         TYPE BINUCLEAR CLUSTER DOMAIN.
DR   EMBL; X78344; G559524; -.
DR   EMBL; Z49704; G825546; -.
DR   EMBL; X94215; E214033; -.
DR   PIR; S48234; S48234.
DR   SGD; L0000220; CAT8.
DR   PROSITE; PS00463; ZN2_CY6_FUNGAL_1; 1.
DR   PROSITE; PS50048; ZN2_CY6_FUNGAL_2; 1.
KW   TRANSCRIPTION REGULATION; DNA-BINDING; NUCLEAR
KW   PROTEIN; ZINC; METAL-BINDING;
KW   PHOSPHORYLATION; CARBOHYDRATE METABOLISM; ACTIVATOR.
FT   DNA_BIND 70 97   ZN(2)-CYS(6), FUNGAL-TYPE.
...
SQ   SEQUENCE   1433 AA;  160485 MW;  3E914E7E CRC32;
     MANNNSDRQG LEPRVIRTLG SQALSGPSIS NRTSSSEANP
     HFSKNVKEAM IKTASPTPLS TPIYRIAQAC DRCRSKKTRC
     DGKRPQCSQC AAVGFECRIS DKLLRKAYPK GYTESLEERV
...
```

Figure 4:

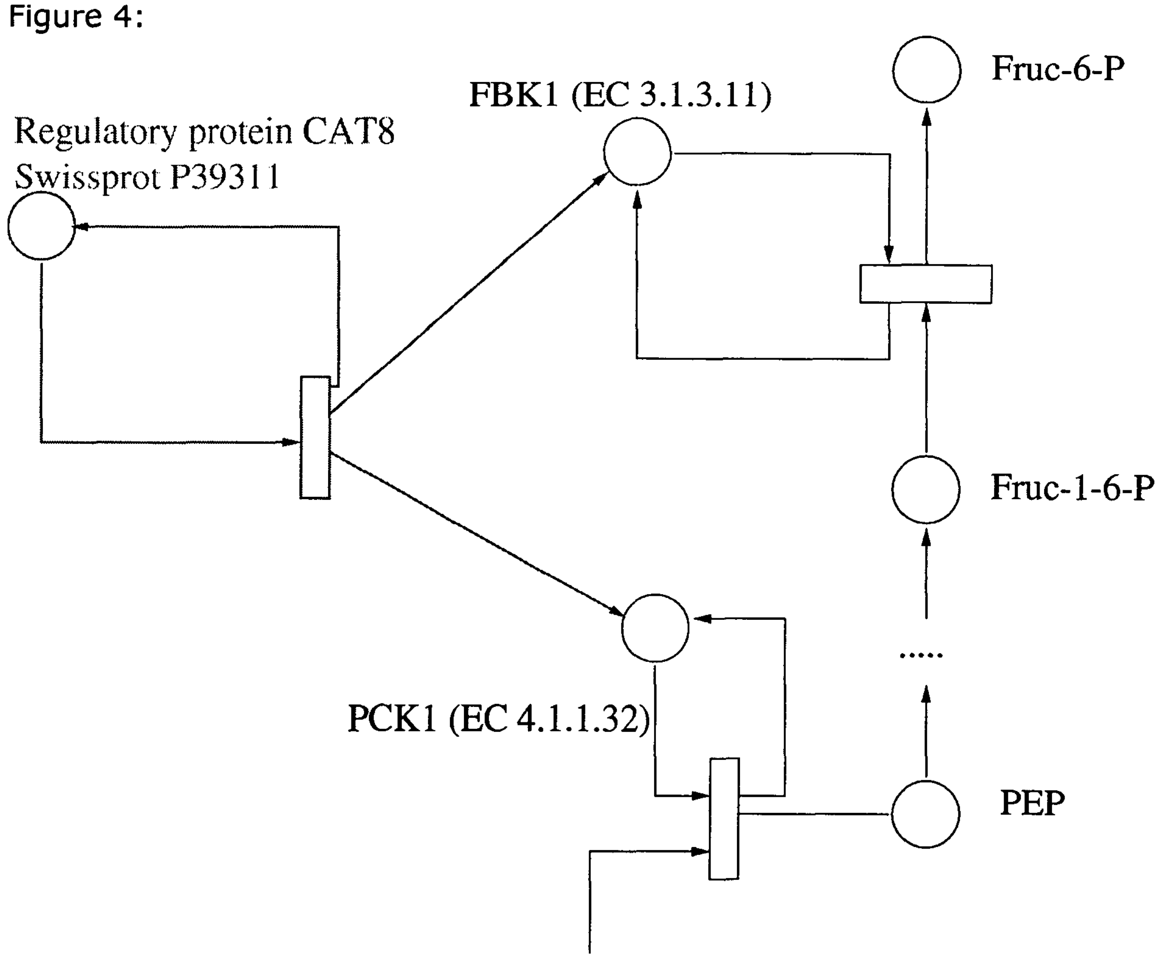
Regulatory protein CAT8
Swissprot P39311
FBK1 (EC 3.1.3.11)
Fruc-6-P
Fruc-1-6-P
.....
PCK1 (EC 4.1.1.32)
PEP

Figure 5:

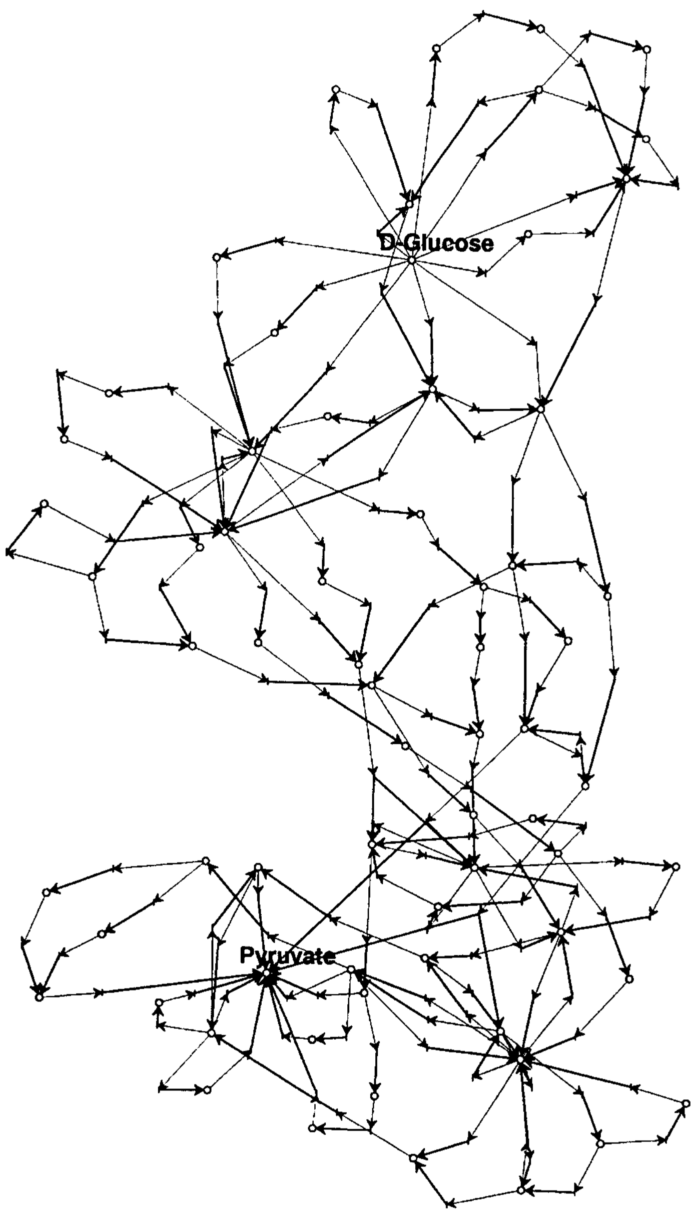

Figure 6:

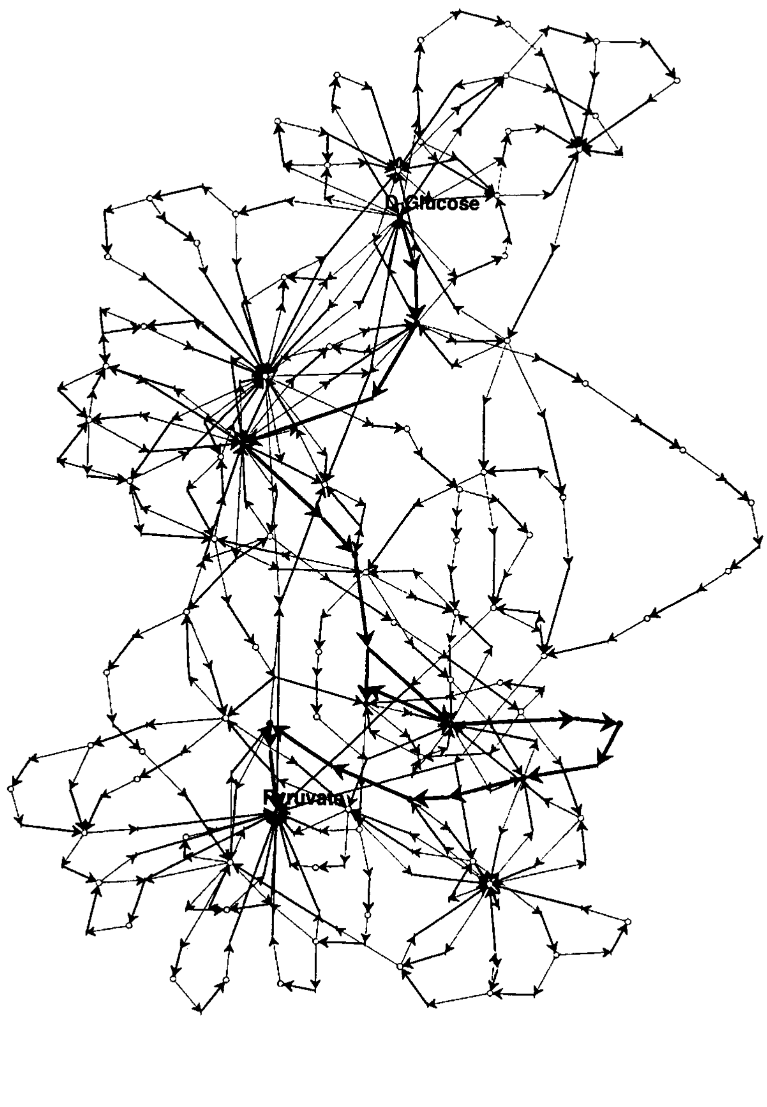

Figure 7:

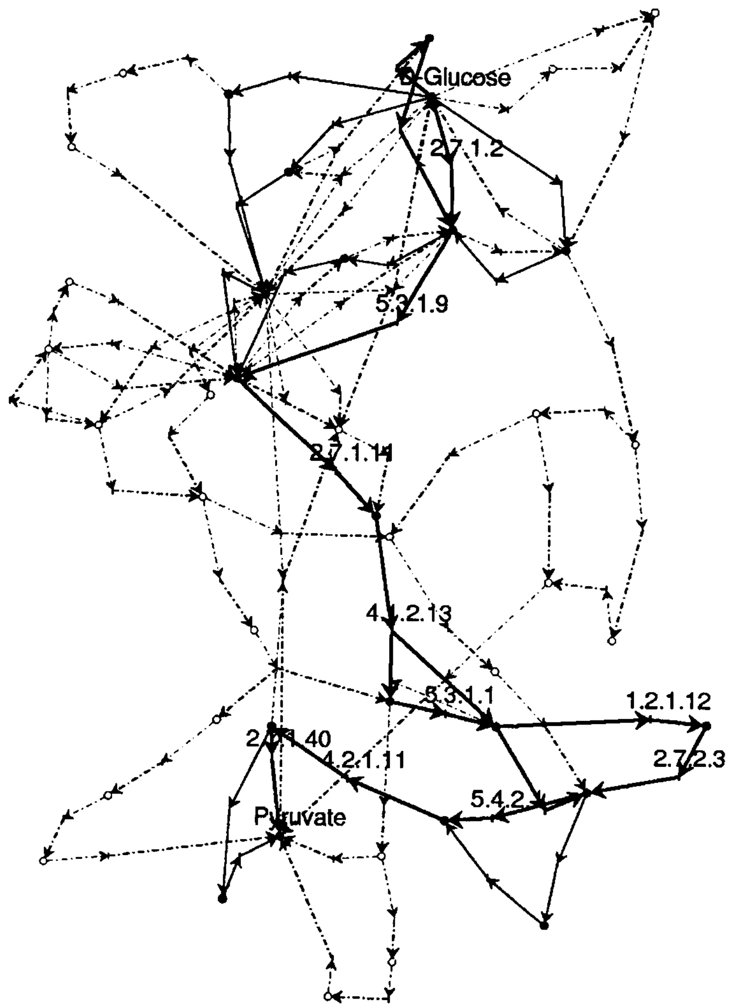
D-Glucose
2.7.1.2
5.3.1.9
2.7.1.11
4.1.2.13
5.3.1.1
1.2.1.12
2.7.2.3
5.4.2.1
4.2.1.11
2.7.1.40
Pyruvate

Figure 8:

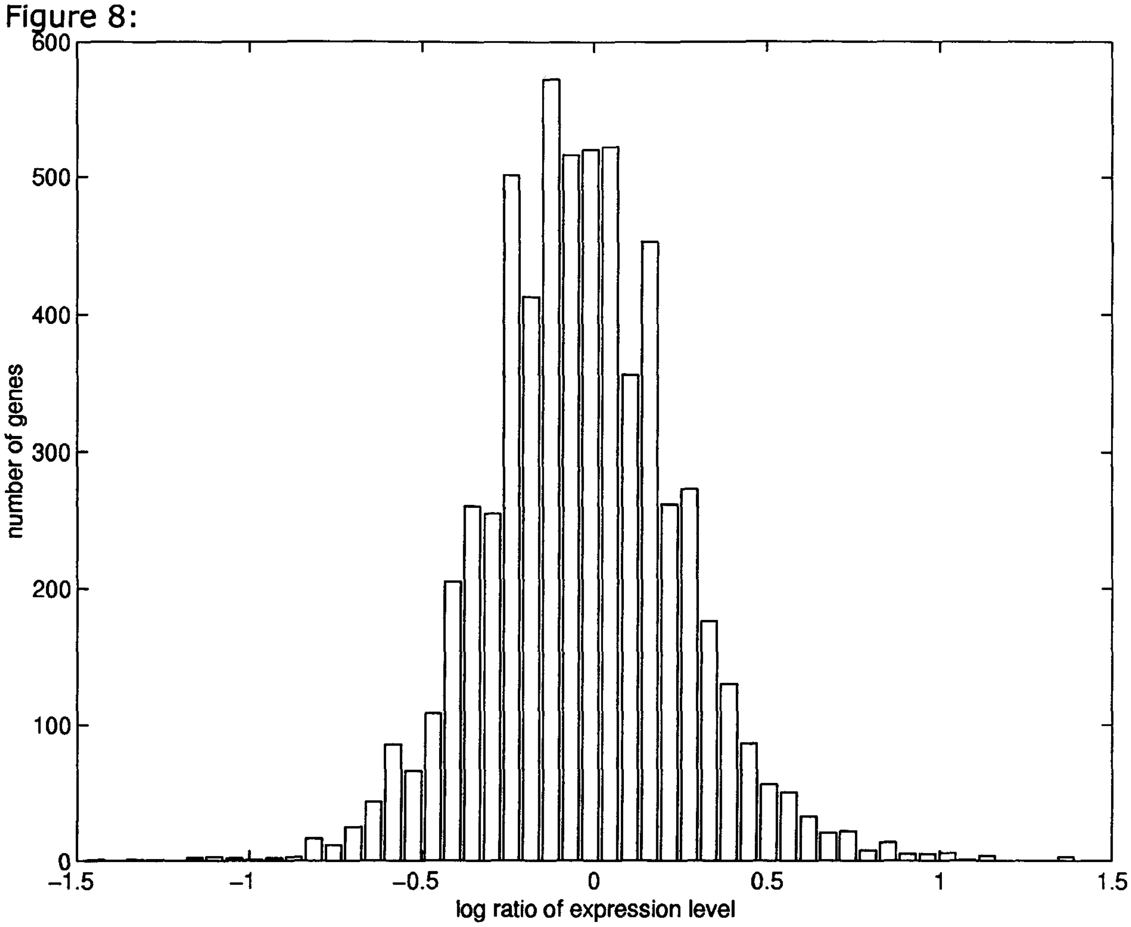
600
500
400
300
200
100
0
number of genes
-1.5
-1
-0.5
0
0.5
1
1.5
log ratio of expression level

Figure 9:

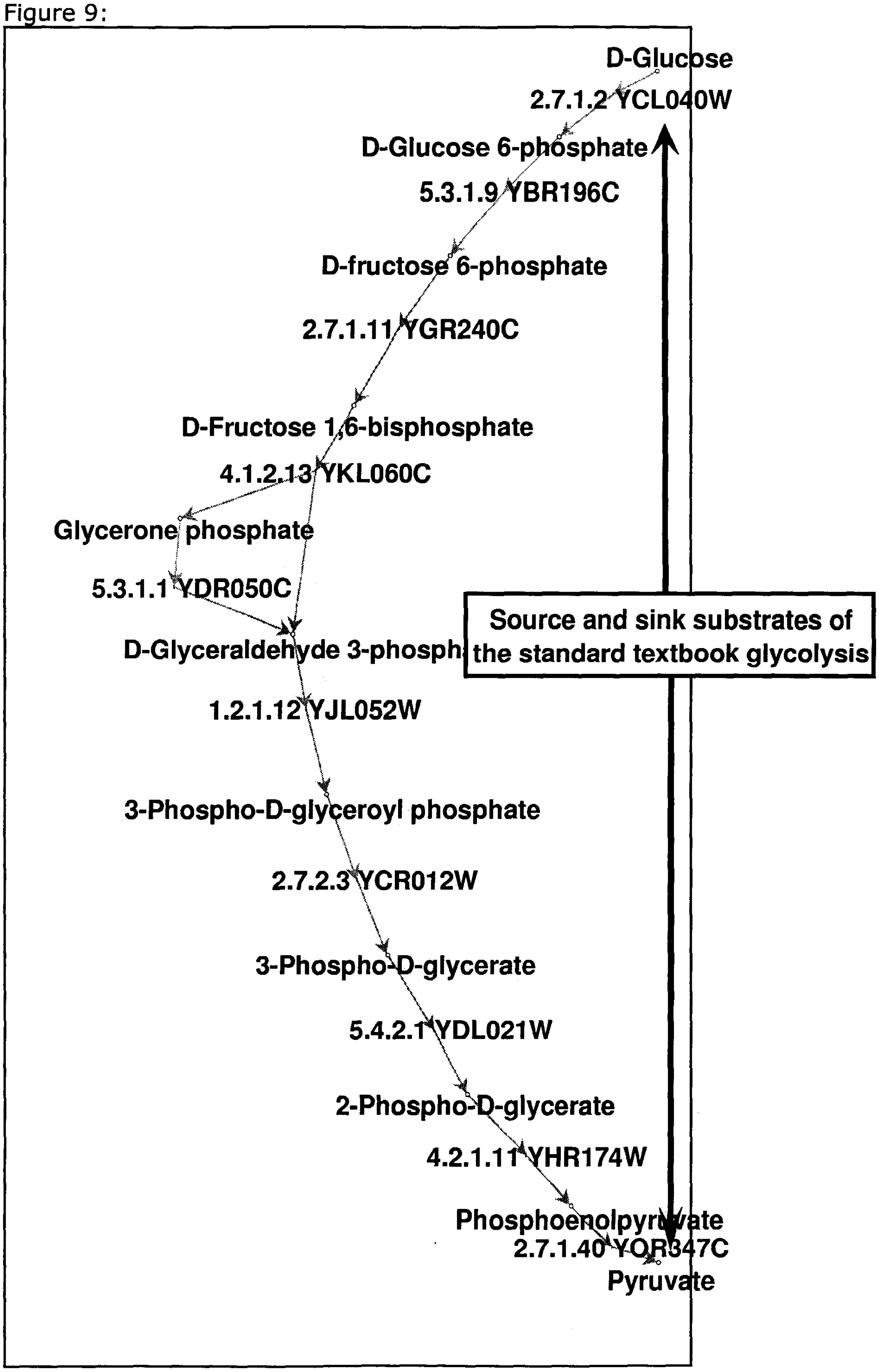

D-Glucose
2.7.1.2 YCL040W
D-Glucose 6-phosphate
5.3.1.9 YBR196C
D-fructose 6-phosphate
2.7.1.11 YGR240C
D-Fructose 1,6-bisphosphate
4.1.2.13 YKL060C
Glycerone phosphate
5.3.1.1 YDR050C
D-Glyceraldehyde 3-phosph
Source and sink substrates of the standard textbook glycolysis
1.2.1.12 YJL052W
3-Phospho-D-glyceroyl phosphate
2.7.2.3 YCR012W
3-Phospho-D-glycerate
5.4.2.1 YDL021W
2-Phospho-D-glycerate
4.2.1.11 YHR174W
Phosphoenolpyruvate
2.7.1.40 YOR347C
Pyruvate

Figure 10:

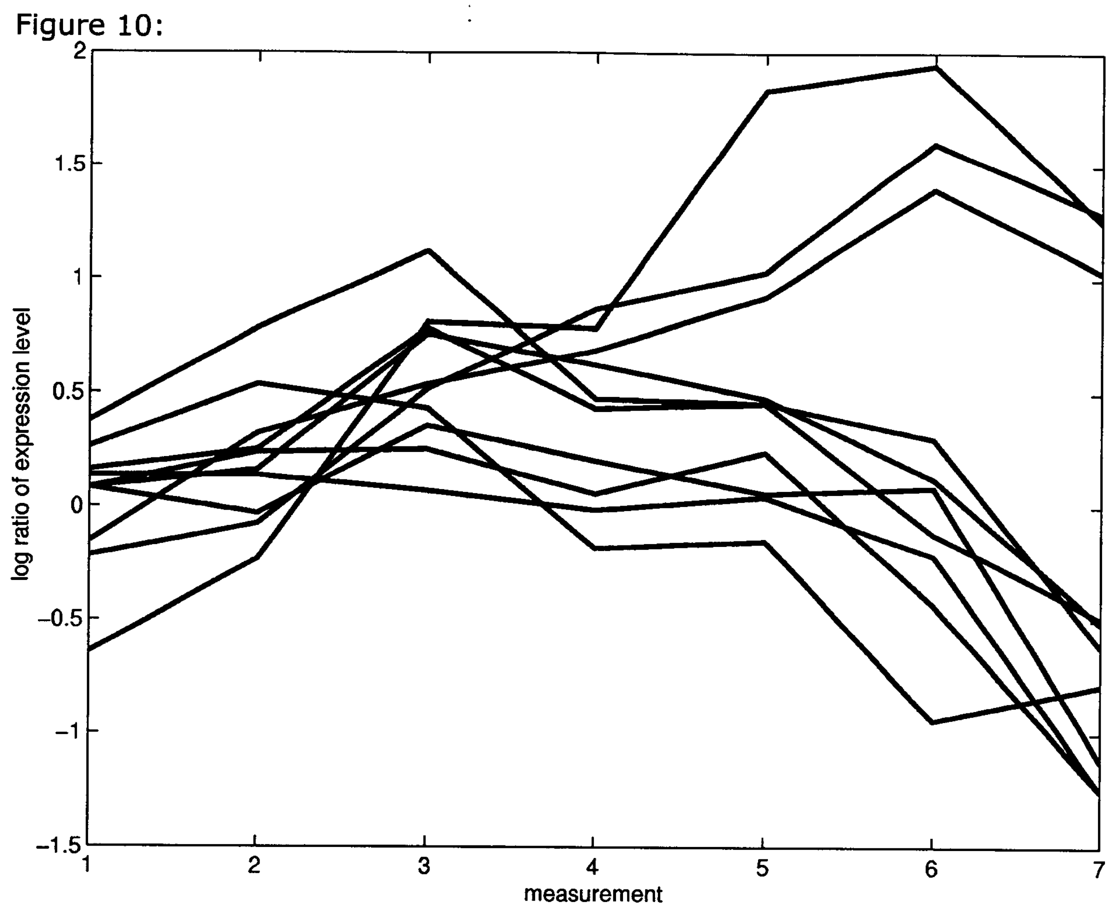
2
1.5
1
0.5
0
−0.5
−1
−1.5
log ratio of expression level
1
2
3
4
5
6
7
measurement

Figure 11:

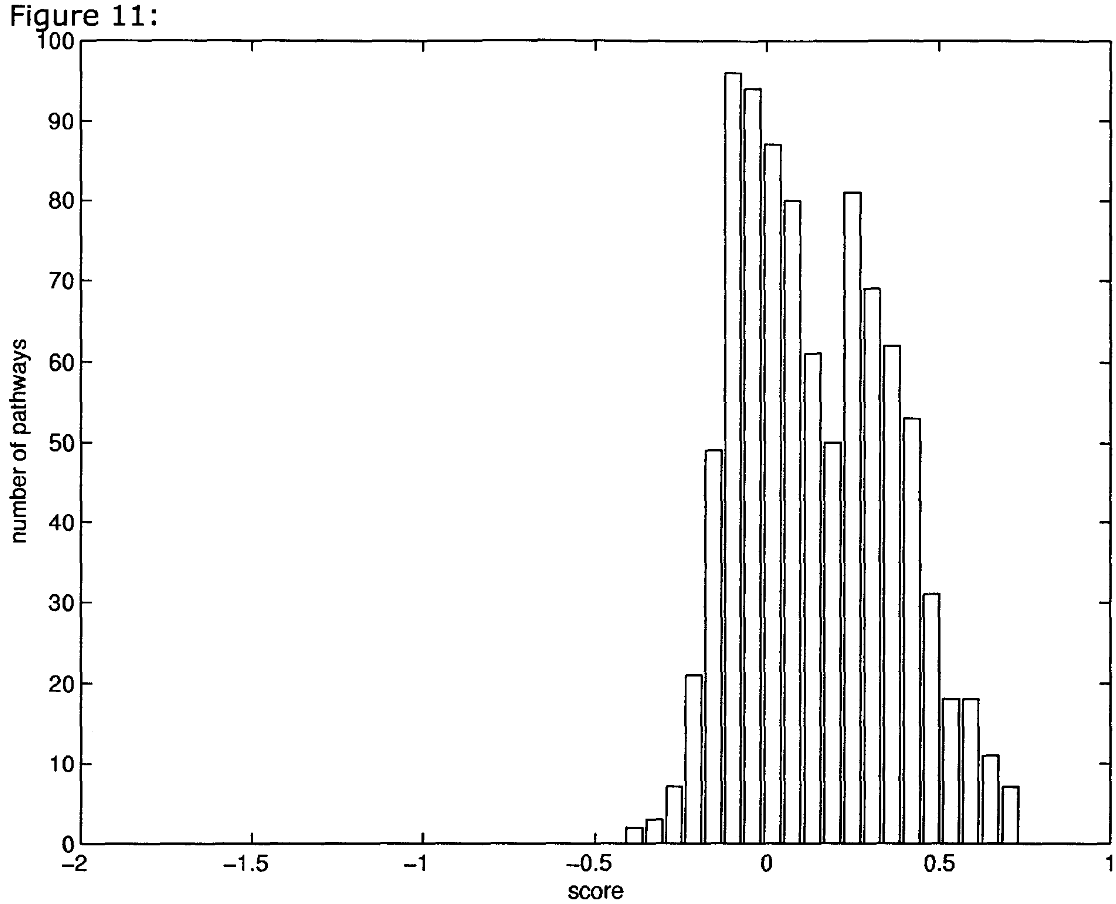
100
90
80
70
60
50
40
30
20
10
0
number of pathways
-2
-1.5
-1
-0.5
0
0.5
1
score

Figure 12:

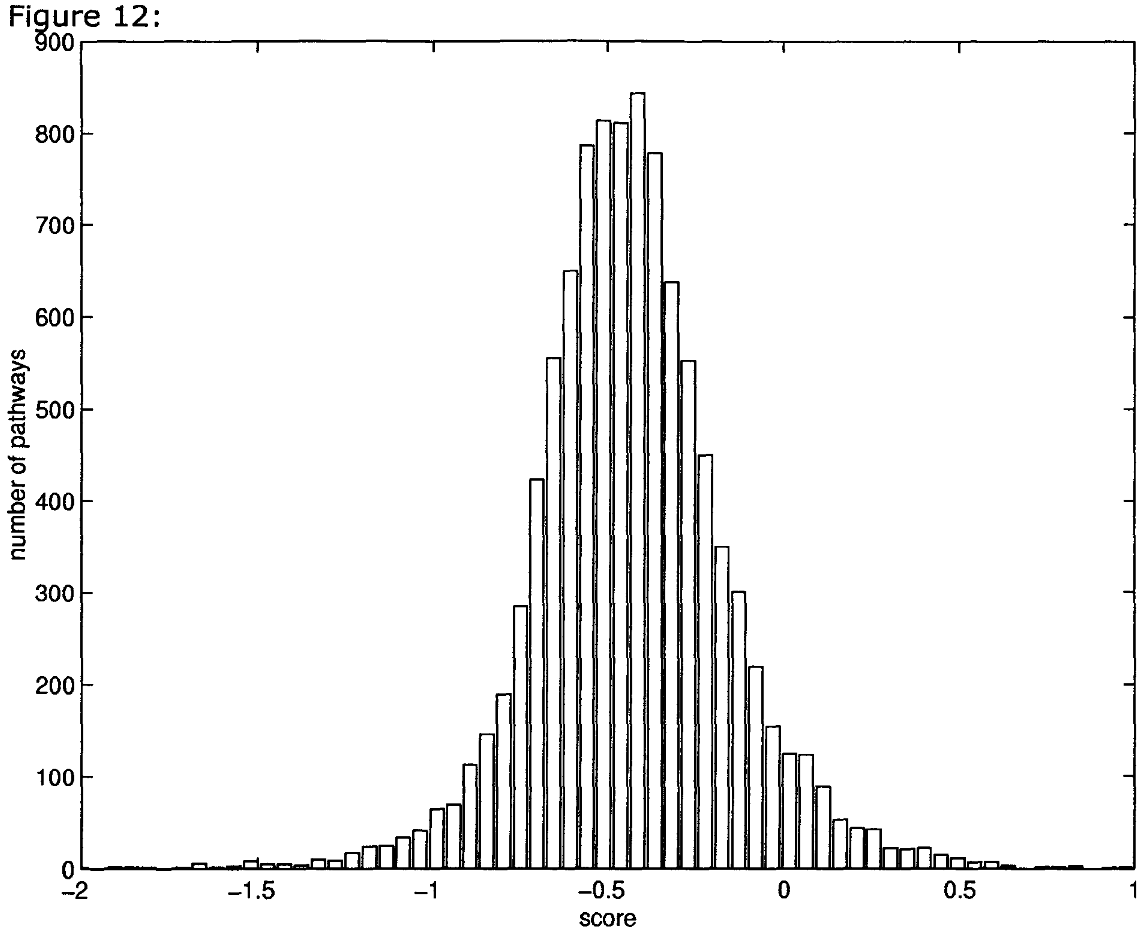
900
800
700
600
500
400
300
200
100
0
number of pathways
−2
−1.5
−1
−0.5
0
0.5
1
score

Figure 13:

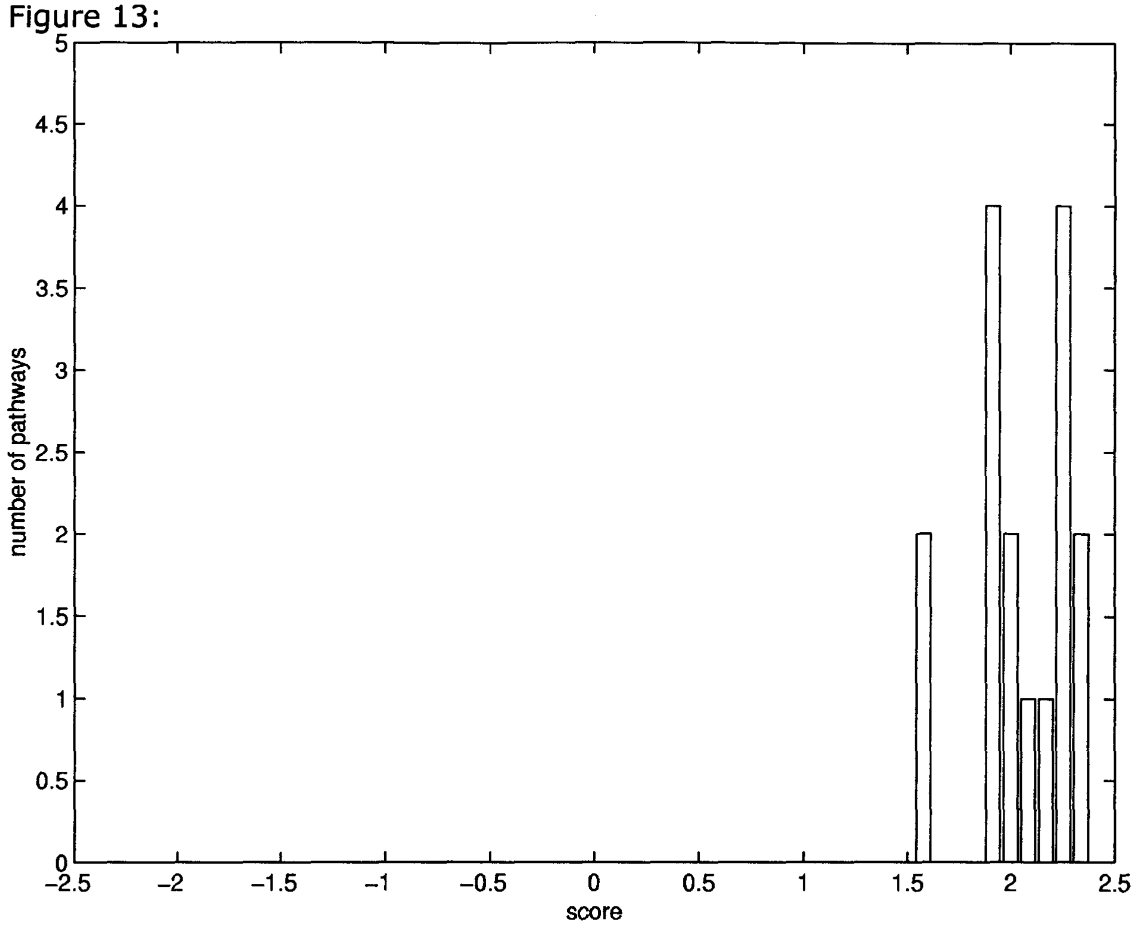
number of pathways
score
0
0.5
1
1.5
2
2.5
3
3.5
4
4.5
5
−2.5
−2
−1.5
−1
−0.5
0
0.5
1
1.5
2
2.5

Figure 14:

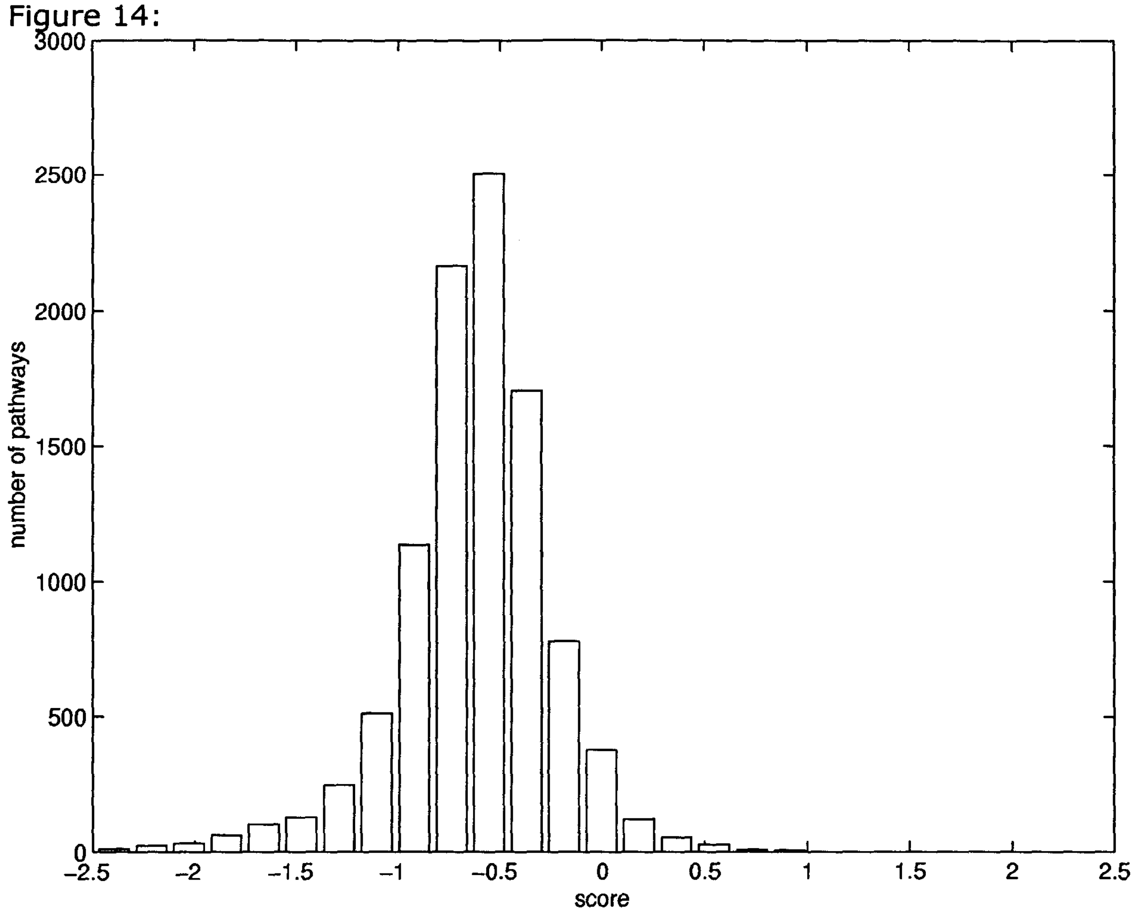
3000
2500
2000
1500
1000
500
0
number of pathways
−2.5
−2
−1.5
−1
−0.5
0
0.5
1
1.5
2
2.5
score

Figure 15:

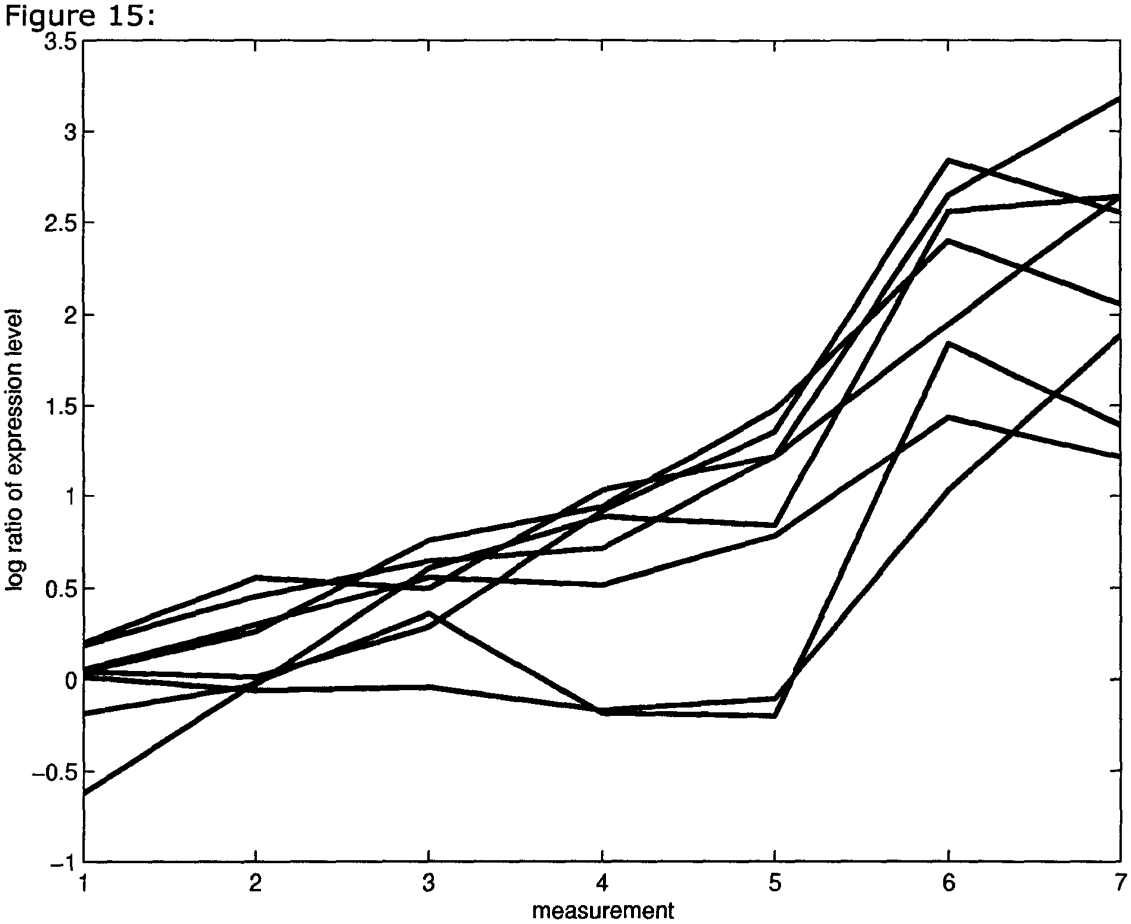
3.5
3
2.5
2
1.5
1
0.5
0
−0.5
−1
log ratio of expression level
1
2
3
4
5
6
7
measurement

Table 1:

| gene (yeast ORF ID) | t1 | t2 | t3 | t4 | t5 | t6 | t7 |
|---|---|---|---|---|---|---|---|
| YCL040W | -0,644 | -0,234 | 0,807 | 0,782 | 1,836 | 1,945 | 1,251 |
| YBR196C | 0,379 | 0,782 | 1,118 | 0,475 | 0,454 | -0,12 | -0,494 |
| YDR050C | 0,084 | -0,029 | 0,356 | 0,202 | 0,057 | 0,084 | -1,12 |
| YGR240C | 0,263 | 0,536 | 0,433 | -0,184 | -0,152 | -0,943 | -0,786 |
| YKL060C | 0,138 | 0,138 | 0,07 | -0,014 | 0,043 | -0,218 | -1,252 |
| YJL052W | 0,163 | 0,251 | 0,782 | 0,433 | 0,454 | 0,299 | -0,621 |
| YCR012W | 0,084 | 0,163 | 0,757 | 0,623 | 0,475 | 0,124 | -0,515 |
| YDL021W | -0,218 | -0,074 | 0,516 | 0,864 | 1,029 | 1,599 | 1,287 |
| YHR174W | 0,084 | 0,239 | 0,251 | 0,057 | 0,239 | -0,434 | -1,252 |
| YOR347C | -0,152 | 0,322 | 0,536 | 0,687 | 0,918 | 1,395 | 1,029 |

Table 2:

| pathway | t1 | t2 | t3 | t4 | t5 | t6 | t7 |
|---|---|---|---|---|---|---|---|
| p - {YCL040W} | 0,092 | 0,259 | 0,535 | 0,349 | 0,391 | 0,198 | -0,414 |
| p - {YBR196C} | -0,022 | 0,146 | 0,501 | 0,383 | 0,544 | 0,428 | -0,220 |
| p - {YDR050C} | 0,011 | 0,236 | 0,586 | 0,414 | 0,588 | 0,405 | -0,150 |
| p - {YGR240C} | -0,009 | 0,173 | 0,577 | 0,457 | 0,612 | 0,519 | -0,187 |
| p - {YKL060C} | 0,005 | 0,217 | 0,617 | 0,438 | 0,590 | 0,439 | -0,136 |
| p - {YJL052W} | 0,002 | 0,205 | 0,538 | 0,388 | 0,544 | 0,381 | -0,206 |
| p - {YCR012W} | 0,011 | 0,215 | 0,541 | 0,367 | 0,542 | 0,401 | -0,218 |
| p - {YDL021W} | 0,044 | 0,241 | 0,568 | 0,340 | 0,480 | 0,237 | -0,418 |
| p - {YHR174W} | 0,011 | 0,206 | 0,597 | 0,430 | 0,568 | 0,463 | -0,136 |
| p - {YOR347C} | 0,037 | 0,197 | 0,566 | 0,360 | 0,493 | 0,260 | -0,389 |

Table 3:

| pathway | t1 | t2 | t3 | t4 | t5 | t6 | t7 |
|---|---|---|---|---|---|---|---|
| p - {YCL040W} | 0,185 | 0,267 | 0,314 | 0,354 | 0,395 | 0,822 | 0,941 |
| p - {YBR196C} | 0,278 | 0,231 | 0,253 | 0,381 | 0,625 | 0,992 | 1,090 |
| p - {YDR050C} | 0,308 | 0,302 | 0,318 | 0,376 | 0,600 | 1,003 | 1,044 |
| p - {YGR240C} | 0,295 | 0,290 | 0,324 | 0,316 | 0,571 | 0,882 | 1,075 |
| p - {YKL060C} | 0,305 | 0,313 | 0,271 | 0,351 | 0,598 | 0,985 | 1,028 |
| p - {YJL052W} | 0,304 | 0,314 | 0,317 | 0,382 | 0,625 | 1,009 | 1,085 |
| p - {YCR012W} | 0,308 | 0,314 | 0,319 | 0,372 | 0,625 | 1,005 | 1,089 |
| p - {YDL021W} | 0,296 | 0,296 | 0,327 | 0,339 | 0,598 | 0,900 | 0,932 |
| p - {YHR174W} | 0,308 | 0,314 | 0,306 | 0,361 | 0,616 | 0,963 | 1,028 |
| p - {YOR347C} | 0,302 | 0,312 | 0,327 | 0,366 | 0,609 | 0,934 | 0,985 |

Table 4:

| | t1 | t2 | t3 | t4 | t5 | t6 | t7 |
|---|---|---|---|---|---|---|---|
| mean_r | -0,089 | -0,035 | 0,116 | -0,197 | -0,242 | 0,089 | -0,202 |
| sd_r | 0,251 | 0,283 | 0,317 | 0,342 | 0,388 | 0,821 | 0,886 |

Table 5:

| gene | t1 | t2 | t3 | t4 | t5 | t6 | t7 | average |
|---|---|---|---|---|---|---|---|---|
| YCL040W | -5,929 | -1,998 | 2,584 | 3,964 | 7,908 | 0,346 | -0,274 | 0,943 |
| YBR196C | 0,870 | 0,401 | 2,250 | 2,797 | 2,496 | -0,319 | 0,078 | 1,225 |
| YDR050C | 0,503 | -0,951 | 0,046 | 0,857 | -0,161 | -0,284 | 0,164 | 0,025 |
| YGR240C | 0,794 | 1,570 | 0,726 | -3,122 | -1,507 | -0,763 | 0,126 | -0,311 |
| YKL060C | 0,593 | 0,391 | -3,017 | -1,095 | -0,250 | -0,339 | 0,163 | -0,508 |
| YJL052W | 0,636 | 1,039 | 2,518 | 2,629 | 2,496 | 0,158 | 0,099 | 1,368 |
| YCR012W | 0,503 | 0,585 | 2,447 | 3,396 | 2,649 | -0,210 | 0,081 | 1,350 |
| YDL021W | -0,481 | -1,131 | 1,440 | 4,160 | 5,825 | 0,682 | -0,318 | 1,454 |
| YHR174W | 0,503 | 1,012 | -0,957 | -0,415 | 1,013 | -0,398 | 0,163 | 0,132 |
| YOR347C | -0,411 | 1,199 | 1,611 | 3,642 | 5,153 | 0,699 | -0,093 | 1,686 |
| average | -0,242 | 0,212 | 0,965 | 1,681 | 2,562 | -0,043 | 0,019 | 0,736 |

European Patent Office

# EUROPEAN SEARCH REPORT

Application Number
EP 00 11 1352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X,D | KÜFFNER R ET AL: "Pathway Analysis in Metablic Databases via Differential Metabolic Display (DMD)" GCB99 CONFERENCE PROCEEDINGS, 'Online! 1999, pages 141-147, XP002153929 Retrieved from the Internet: <URL:http://www.bioinfo.de/isb/gcb99/talks/kueffner/main.html> 'retrieved on 2000-11-23! * the whole document * --- | 1-15 | G06F19/00 |
| X | DIXON D A ET AL: "Enabling the Silicon Cell: Development of Models of Cell-Signaling Pathways and Networks" THEROY, MODELING, AND SIMULATION 1999 ANNUAL REPORT, WILLIAM R. WILEY ENVIRONMENTAL MOLECULAR SCIENCES LABORATORY, 'Online! 28 March 2000 (2000-03-28), XP002153930 Retrieved from the Internet: <URL:http://www.emsl.pnl.gov:2080/docs/tms/annual_report1999/1619b-4q.html> 'retrieved on 2000-11-24! * the whole document * --- | 1,8-15 | TECHNICAL FIELDS SEARCHED (Int.Cl.7) G06F |
| A | LEMIEUX B ET AL: "OVERVIEW OF DNA CHIP TECHNOLOGY" MOLECULAR BREEDING: NEW STRATEGIES IN PLANT IMPROVEMENT,KLUWER ACADEMIC PUBLISHERS,NL, vol. 4, 1998, pages 277-289, XP000915221 ISSN: 1380-3743 * page 284, left-hand column, paragraph 3 * --- -/-- | 1-15 | |

The present search report has been drawn up for all claims

| Place of search | Date of completion of the search | Examiner |
|---|---|---|
| THE HAGUE | 27 November 2000 | Filloy García, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 11 1352

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HOFESTÄDT R AND THELEN S: "Quantitative Modeling of Biochemical Networks" IN SILICO BIOLOGY 1, 0006, 'Online! 1998, XP002153931 Retrieved from the Internet: <URL:http://www.bioinfo.de/isb/1998/01/0006/> 'retrieved on 2000-11-24! * abstract; conclusion * | 1-15 | |
| A | WO 96 33703 A (O DAY CHRISTINE L ;SCHWARTZ DENNIS E (US); ORIDIGM CORP (US); VERM) 31 October 1996 (1996-10-31) * page 82, line 22 - line 32 * | 1,11 | |
| A | LEMMEN C ET AL: "Application of parameter optimization to molecular comparison problems" PACIFIC SYMPOSIUM ON BIOCOMPUTING, 'Online! 1999, pages 482-493, XP002153932 Retrieved from the Internet: <URL:http://www.smi.stanford.edu/projects/helix/psb99/Lemmen.pdf> 'retrieved on 2000-11-24! * abstract * | 2-7 | TECHNICAL FIELDS SEARCHED (Int.Cl.7) |

The present search report has been drawn up for all claims

| Place of search | Date of completion of the search | Examiner |
|---|---|---|
| THE HAGUE | 27 November 2000 | Filloy García, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 11 1352

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2000

| Patent document cited in search report | Publication date | Patent family member(s) | Publication date |
|---|---|---|---|
| WO 9633703 A | 31-10-1996 | US 6020139 A | 01-02-2000 |
| | | AU 721067 B | 22-06-2000 |
| | | AU 5715696 A | 18-11-1996 |
| | | CA 2217696 A | 31-10-1996 |
| | | EP 0824345 A | 25-02-1998 |
| | | JP 11506426 T | 08-06-1999 |

EPO FORM P0459

For more details about this annex : see Official Journal of the European Patent Office, No. 12/82